# EUROPEAN PATENT APPLICATION

(11) **EP 2 128 250 A1**
(43) Date of publication of application: **02.12.2009**
(21) Application number: 08720363.4
(22) Date of filing: 07.03.2008
(51) Int. Cl.: C12N 15/00, C12N 9/00, C12N 9/16, C12N 15/09

(54) **COMPOSITION FOR CLEAVING AND/OR CONNECTING SINGLE STRAND DNA**

(30) Priority: 07.03.2007 JP 2007056550
(71) Applicant: Waseda University, Tokyo 169-8050 (JP)
(72) Inventor: KURUMIZAKA, Hitoshi, Tokyo 169-8555 (JP); TAKAKU, Motoki, Tokyo 169-8555 (JP); MACHIDA, Shinichi, Tokyo 169-8555 (JP)
(74) Representative: Docherty, Robert Charles
(86) International application number: PCT/JP2008/000477
(87) International publication number: WO 2008/108103

(57) **Abstract**

It is an object of the present invention to provide a composition for catalyzing the cleavage of a single-stranded DNA and the binding of such single-stranded DNA. The present invention provides a composition for cleaving a single-stranded DNA and/or binding the 5'-terminus of such single-stranded DNA to the 3'-terminus thereof, which comprises an Evl protein. Moreover, the present invention also provides a composition for cleaving a single-stranded DNA and/or binding the 5'-terminus of such single-stranded DNA to the 3'-terminus thereof, which further comprises a Rad51B protein and/or a DNA topoisomerase type I protein, as well as the Evl protein.

### Sequence Listing

SEQUENCE LISTING

<110>  Waseda University

<120>  The novel Rad51B-interacting protein, Evl

<130>  PC0189WAS

<150>  JP2007-056550
<151>  2007-03-07

<160>  20

<170>  PatentIn version 3.1

<210>  1
<211>  1257
<212>  DNA
<213>  Homo sapiens

<400>  1
atggccacaa gtgaacagag tatctgccaa gcccgggctt ccgtgatggt ctacgatgac      60

accagtaaga aatgggtacc aatcaaacct ggccagcagg gattcagccg gatcaacatc     120

taccacaaca ctgccagcaa caccttcaga gtcgttggag tcaagttgca ggatcagcag     180

gttgtgatca attattcaat cgtgaaaggg ctgaagtaca atcaggccac gccaaccttc     240

caccagtggc gagatgcccg ccaggtctac ggcttaaact ttgcaagtaa agaagaggca     300

accacgttct ccaatgcaat gctgtttgcc ctgaacatca tgaattccca agaaggaggc     360

ccctccagcc agcgtcaggt gcagaatggc ccctctcctg atgagatgga catccagaga     420

agacaagtga tggagcagca ccagcagcag cgtcaggaat ctctagaaag aagaacctcg     480

gccacagggc ccatcctccc accaggacat ccttcatctg cagccagcgc ccccgtctca     540

tgtagtgggc ctccaccgcc ccccccaccc ccagtcccac ctccacccac tggggctacc     600

ccacctcccc cacccccact gccagccgga ggagcccagg ggtccagcca cgacgagagc     660

tccatgtcag gactggccgc tgccatagct ggggccaagc tgagaagagt ccaacggcca     720

gaagacgcat ctggaggctc cagtcccagt gggacctcaa agtccgatgc caaccgggca     780

agcagcgggg gtggcggagg aggcctcatg gaggaaatga acaaactgct ggccaagagg     840

agaaaagcag cctcccagtc agacaagcca gccgagaaga aggaagatga aagccaaatg     900

gaagatccta gtacctcccc ctctccgggg acccgagcag ccagccagcc acctaactcc     960

tcagaggctg gccggaagcc ctgggagcgg agcaactcgg tggagaagcc tgtgtcctcg    1020

attctgtcca gaaccccgtc tgtggcaaag agccccgaag ctaagagccc ccttcagtcg 1080

cagcctcact ctaggatgaa gcctgctggg agcgtgaatg acatggccct ggatgccttc 1140

gacttggacc ggatgaagca ggagatccta gaggaggtgg tgagagagct ccacaaggtg 1200

aaggaggaga tcatcgacgc catcaggcag gagctgagtg ggatcagcac cacgtaa 1257

<210> 2
<211> 418
<212> PRT
<213> Homo sapiens

<400> 2

Met Ala Thr Ser Glu Gln Ser Ile Cys Gln Ala Arg Ala Ser Val Met
1               5                   10                  15

Val Tyr Asp Asp Thr Ser Lys Lys Trp Val Pro Ile Lys Pro Gly Gln
            20                  25                  30

Gln Gly Phe Ser Arg Ile Asn Ile Tyr His Asn Thr Ala Ser Asn Thr
        35                  40                  45

Phe Arg Val Val Gly Val Lys Leu Gln Asp Gln Gln Val Val Ile Asn
    50                  55                  60

Tyr Ser Ile Val Lys Gly Leu Lys Tyr Asn Gln Ala Thr Pro Thr Phe
65                  70                  75                  80

His Gln Trp Arg Asp Ala Arg Gln Val Tyr Gly Leu Asn Phe Ala Ser
                85                  90                  95

Lys Glu Glu Ala Thr Thr Phe Ser Asn Ala Met Leu Phe Ala Leu Asn
            100                 105                 110

Ile Met Asn Ser Gln Glu Gly Gly Pro Ser Ser Gln Arg Gln Val Gln
        115                 120                 125

Asn Gly Pro Ser Pro Asp Glu Met Asp Ile Gln Arg Arg Gln Val Met
    130                 135                 140

Glu Gln His Gln Gln Gln Arg Gln Glu Ser Leu Glu Arg Arg Thr Ser

145                 150                 155                 160

Ala Thr Gly Pro Ile Leu Pro Pro Gly His Pro Ser Ser Ala Ala Ser
                165                 170                 175

Ala Pro Val Ser Cys Ser Gly Pro Pro Pro Pro Pro Pro Pro Pro Val
            180                 185                 190

Pro Pro Pro Pro Thr Gly Ala Thr Pro Pro Pro Pro Pro Pro Leu Pro
        195                 200                 205

Ala Gly Gly Ala Gln Gly Ser Ser His Asp Glu Ser Ser Met Ser Gly
    210                 215                 220

Leu Ala Ala Ala Ile Ala Gly Ala Lys Leu Arg Arg Val Gln Arg Pro
225                 230                 235                 240

Glu Asp Ala Ser Gly Gly Ser Ser Pro Ser Gly Thr Ser Lys Ser Asp
                245                 250                 255

Ala Asn Arg Ala Ser Ser Gly Gly Gly Gly Gly Gly Leu Met Glu Glu
            260                 265                 270

Met Asn Lys Leu Leu Ala Lys Arg Arg Lys Ala Ala Ser Gln Ser Asp
        275                 280                 285

Lys Pro Ala Glu Lys Lys Glu Asp Glu Ser Gln Met Glu Asp Pro Ser
    290                 295                 300

Thr Ser Pro Ser Pro Gly Thr Arg Ala Ala Ser Gln Pro Pro Asn Ser
305                 310                 315                 320

Ser Glu Ala Gly Arg Lys Pro Trp Glu Arg Ser Asn Ser Val Glu Lys
                325                 330                 335

Pro Val Ser Ser Ile Leu Ser Arg Thr Pro Ser Val Ala Lys Ser Pro
            340                 345                 350

Glu Ala Lys Ser Pro Leu Gln Ser Gln Pro His Ser Arg Met Lys Pro
        355                 360                 365

Ala Gly Ser Val Asn Asp Met Ala Leu Asp Ala Phe Asp Leu Asp Arg
    370                 375                 380

Met Lys Gln Glu Ile Leu Glu Glu Val Val Arg Glu Leu His Lys Val
385                 390                 395                 400

Lys Glu Glu Ile Ile Asp Ala Ile Arg Gln Glu Leu Ser Gly Ile Ser
                405                 410                 415

Thr Thr

<210>  3
<211>  1091
<212>  DNA
<213>  Homo sapiens

<400>  3
tgcaggatca gcaggttgtg atcaattatt caatcgtgaa agggctgaag tacaatcagg      60

ccacgccaac cttccaccag tggcgagatg cccgccaggt ctacggctta aactttgcaa     120

gtaaagaaga ggcaaccaca ttctccaatg caatgctgtt tgccctgaac atcatgaatt     180

cccaagaagg aggcccctcc agccagcgtc aggtgcagaa tggcccctct cctgatgaga     240

tggacatcca gagaagacaa gtgatggagc agcaccagca gcagcgtcag gaatctctag     300

aaagaagaac ctcggccaca gggcccatcc tcccaccagg acatccttca tctgcagcca     360

gcgcccccgt ctcatgtagt gggcctccac cgcccccccc acccccagtc ccacctccac     420

ccactggggc taccccacct cccccacccc cactgccagc cggaggagcc caggggtcca     480

gccacgacga gagctccatg tcaggactgg ccgctgccat agctggggcc aagctgagaa     540

gagtccaacg gccagaagac gcatctggag gctccagtcc cagtgggacc tcaaagtccg     600

atgccaaccg ggcaagcagc gggggtggcg gaggaggcct catggaggaa atgaacaaac     660

tgctggccaa gaggagaaaa gcagcctccc agtcagacaa gccagccgag aagaaggaag     720

atgaaagcca aatggaagat cctagtacct ccccctctcc ggggacccga gcagccagcc     780

agccacctaa ctcctcagag gctggccgga agccctggga gcggagcaac tcggtggaga     840

agcctgtgtc ctcgattctg tccagaaccc cgtctgtggc aaagagcccc gaagctaaga     900

gcccccttca gtcgcagcct cactctagga tgaagcctgc tgggagcgtg aatgacatgg     960

ccctggatgc cttcgacttg gaccggatga agcaggagat cctagaggag gtggtgagag 1020

agctccacaa ggtgaaggag gagatcatcg acgccatcag gcaggagctg agtgggatca 1080

gcaccacgta a 1091

<210> 4
<211> 362
<212> PRT
<213> Homo sapiens

<400> 4

Gln Asp Gln Gln Val Val Ile Asn Tyr Ser Ile Val Lys Gly Leu Lys
1               5                   10                  15

Tyr Asn Gln Ala Thr Pro Thr Phe His Gln Trp Arg Asp Ala Arg Gln
            20                  25                  30

Val Tyr Gly Leu Asn Phe Ala Ser Lys Glu Glu Ala Thr Thr Phe Ser
        35                  40                  45

Asn Ala Met Leu Phe Ala Leu Asn Ile Met Asn Ser Gln Glu Gly Gly
    50                  55                  60

Pro Ser Ser Gln Arg Gln Val Gln Asn Gly Pro Ser Pro Asp Glu Met
65                  70                  75                  80

Asp Ile Gln Arg Arg Gln Val Met Glu Gln His Gln Gln Gln Arg Gln
                85                  90                  95

Glu Ser Leu Glu Arg Arg Thr Ser Ala Thr Gly Pro Ile Leu Pro Pro
            100                 105                 110

Gly His Pro Ser Ser Ala Ala Ser Ala Pro Val Ser Cys Ser Gly Pro
        115                 120                 125

Pro Pro Pro Pro Pro Pro Pro Val Pro Pro Pro Pro Thr Gly Ala Thr
    130                 135                 140

Pro Pro Pro Pro Pro Pro Leu Pro Ala Gly Gly Ala Gln Gly Ser Ser
145                 150                 155                 160

His Asp Glu Ser Ser Met Ser Gly Leu Ala Ala Ala Ile Ala Gly Ala
                165                 170                 175

Lys Leu Arg Arg Val Gln Arg Pro Glu Asp Ala Ser Gly Gly Ser Ser
            180                 185                 190

Pro Ser Gly Thr Ser Lys Ser Asp Ala Asn Arg Ala Ser Ser Gly Gly
        195                 200                 205

Gly Gly Gly Gly Leu Met Glu Glu Met Asn Lys Leu Leu Ala Lys Arg
    210                 215                 220

Arg Lys Ala Ala Ser Gln Ser Asp Lys Pro Ala Glu Lys Lys Glu Asp
225                 230                 235                 240

Glu Ser Gln Met Glu Asp Pro Ser Thr Ser Pro Ser Pro Gly Thr Arg
                245                 250                 255

Ala Ala Ser Gln Pro Pro Asn Ser Ser Glu Ala Gly Arg Lys Pro Trp
            260                 265                 270

Glu Arg Ser Asn Ser Val Glu Lys Pro Val Ser Ser Ile Leu Ser Arg
        275                 280                 285

Thr Pro Ser Val Ala Lys Ser Pro Glu Ala Lys Ser Pro Leu Gln Ser
    290                 295                 300

Gln Pro His Ser Arg Met Lys Pro Ala Gly Ser Val Asn Asp Met Ala
305                 310                 315                 320

Leu Asp Ala Phe Asp Leu Asp Arg Met Lys Gln Glu Ile Leu Glu Glu
                325                 330                 335

Val Val Arg Glu Leu His Lys Val Lys Glu Glu Ile Ile Asp Ala Ile
            340                 345                 350

Arg Gln Glu Leu Ser Gly Ile Ser Thr Thr
        355                 360

<210>  5

<211>  1251
<212>  DNA
<213>  Homo sapiens

<400>  5
atgagtgaac agagtatctg ccaagcccgg gcttccgtga tggtctacga tgacaccagt      60

aagaaatggg taccaatcaa acctggccag cagggattca gccggatcaa catctaccac     120

aacactgcca gcaacacctt cagagtcgtt ggagtcaagt tgcaggatca gcaggttgtg     180

atcaattatt caatcgtgaa agggctgaag tacaatcagg ccacgccaac cttccaccag     240

tggcgagatg cccgccaggt ctacggctta aactttgcaa gtaaagaaga ggcaaccacg     300

ttctccaatg caatgctgtt tgccctgaac atcatgaatt cccaagaagg aggcccctcc     360

agccagcgtc aggtgcagaa tggcccctct cctgatgaga tggacatcca gagaagacaa     420

gtgatggagc agcaccagca gcagcgtcag gaatctctag aaagaagaac ctcggccaca     480

gggcccatcc tcccaccagg acatccttca tctgcagcca gcgcccccgt ctcatgtagt     540

gggcctccac cgcccccccc acccccagtc ccacctccac ccactggggc taccccacct     600

cccccacccc cactgccagc cggaggagcc caggggtcca gccacgacga gagctccatg     660

tcaggactgg ccgctgccat agctggggcc aagctgagaa gagtccaacg gccagaagac     720

gcatctggag gctccagtcc cagtgggacc tcaaagtccg atgccaaccg ggcaagcagc     780

gggggtggcg gaggaggcct catggaggaa atgaacaaac tgctggccaa gaggagaaaa     840

gcagcctccc agtcagacaa gccagccgag aagaaggaag atgaaagcca aatggaagat     900

cctagtacct ccccctctcc ggggacccga gcagccagcc agccacctaa ctcctcagag     960

gctggccgga agccctggga gcggagcaac tcggtggaga agcctgtgtc ctcgattctg    1020

tccagaaccc cgtctgtggc aaagagcccc gaagctaaga gcccccttca gtcgcagcct    1080

cactctagga tgaagcctgc tgggagcgtg aatgacatgg ccctggatgc cttcgacttg    1140

gaccggatga agcaggagat cctagaggag gtggtgagag agctccacaa ggtgaaggag    1200

gagatcatcg acgccatcag gcaggagctg agtgggatca gcaccacgta a             1251

<210>  6
<211>  416
<212>  PRT
<213>  Homo sapiens

<400>  6

Met Ser Glu Gln Ser Ile Cys Gln Ala Arg Ala Ser Val Met Val Tyr
1               5                   10                  15

Asp Asp Thr Ser Lys Lys Trp Val Pro Ile Lys Pro Gly Gln Gln Gly
            20                  25                  30

Phe Ser Arg Ile Asn Ile Tyr His Asn Thr Ala Ser Asn Thr Phe Arg
        35                  40                  45

Val Val Gly Val Lys Leu Gln Asp Gln Gln Val Val Ile Asn Tyr Ser
    50                  55                  60

Ile Val Lys Gly Leu Lys Tyr Asn Gln Ala Thr Pro Thr Phe His Gln
65                  70                  75                  80

Trp Arg Asp Ala Arg Gln Val Tyr Gly Leu Asn Phe Ala Ser Lys Glu
                85                  90                  95

Glu Ala Thr Thr Phe Ser Asn Ala Met Leu Phe Ala Leu Asn Ile Met
            100                 105                 110

Asn Ser Gln Glu Gly Gly Pro Ser Ser Gln Arg Gln Val Gln Asn Gly
        115                 120                 125

Pro Ser Pro Asp Glu Met Asp Ile Gln Arg Arg Gln Val Met Glu Gln
    130                 135                 140

His Gln Gln Gln Arg Gln Glu Ser Leu Glu Arg Arg Thr Ser Ala Thr
145                 150                 155                 160

Gly Pro Ile Leu Pro Pro Gly His Pro Ser Ser Ala Ala Ser Ala Pro
                165                 170                 175

Val Ser Cys Ser Gly Pro Pro Pro Pro Pro Pro Pro Pro Val Pro Pro
            180                 185                 190

Pro Pro Thr Gly Ala Thr Pro Pro Pro Pro Pro Pro Leu Pro Ala Gly
        195                 200                 205

Gly Ala Gln Gly Ser Ser His Asp Glu Ser Ser Met Ser Gly Leu Ala

210                 215                 220

Ala Ala Ile Ala Gly Ala Lys Leu Arg Arg Val Gln Arg Pro Glu Asp
225                 230                 235                 240

Ala Ser Gly Gly Ser Ser Pro Ser Gly Thr Ser Lys Ser Asp Ala Asn
                245                 250                 255

Arg Ala Ser Ser Gly Gly Gly Gly Gly Gly Leu Met Glu Glu Met Asn
            260                 265                 270

Lys Leu Leu Ala Lys Arg Arg Lys Ala Ala Ser Gln Ser Asp Lys Pro
        275                 280                 285

Ala Glu Lys Lys Glu Asp Glu Ser Gln Met Glu Asp Pro Ser Thr Ser
    290                 295                 300

Pro Ser Pro Gly Thr Arg Ala Ala Ser Gln Pro Pro Asn Ser Ser Glu
305                 310                 315                 320

Ala Gly Arg Lys Pro Trp Glu Arg Ser Asn Ser Val Glu Lys Pro Val
                325                 330                 335

Ser Ser Ile Leu Ser Arg Thr Pro Ser Val Ala Lys Ser Pro Glu Ala
            340                 345                 350

Lys Ser Pro Leu Gln Ser Gln Pro His Ser Arg Met Lys Pro Ala Gly
        355                 360                 365

Ser Val Asn Asp Met Ala Leu Asp Ala Phe Asp Leu Asp Arg Met Lys
    370                 375                 380

Gln Glu Ile Leu Glu Glu Val Val Arg Glu Leu His Lys Val Lys Glu
385                 390                 395                 400

Glu Ile Ile Asp Ala Ile Arg Gln Glu Leu Ser Gly Ile Ser Thr Thr
                405                 410                 415

<210>  7
<211>  1218
<212>  DNA

<213>  Mus musculus

<400>  7
atgagtgaac agagtatctg ccaagcgcgg gcctccgtga tggtctacga tgacaccagt      60

aagaagtggg taccgatcaa gcctggccag cagggattca gccggatcaa catctaccac     120

aacactgcca gcagcacctt cagagtggtc ggggtcaagc tacaggacca gcaggttgtg     180

atcaattatt caattgttaa agggctgaag tacaatcagg caacacccac cttccatcag     240

tggcgagatg cccgtcaggt ctatggctta aactttgcaa gtaaggaaga agcaaccaca     300

ttctccaatg ccatgctctt tgccctgaac atcatgaatt cccaagaagg aggcccctcc     360

acacagcgtc aggtgcagaa tggcccctct cctgaggaga tggacatcca gagaagacaa     420

gtaatggagc agcagcaccg ccaggagtct ctggagagga gaatctcggc cacagggccc     480

attctccccc ctgggcatcc ctcatcggca gccagcacca ctctctcctg tagtggacct     540

ccacccccgc ctccaccccc agttccacct ccacccacag ggtctactcc cccaccccca     600

cccccactgc cagctggagg agcccagggg accaaccatg atgagagctc tgcatcagga     660

ctggctgctg ctctggcggg agccaagcta aggagggtgc agcggccaga agatgcatct     720

ggaggctcca gtcctagtgg gacttcaaag tccgatgcca accgggcaag cagtggggga     780

ggtggaggag gcctcatgga agaaatgaac aagctgctgg ctaagaggag aaaggcagcc     840

tcccagacag acaagcccgc tgacagaaag gaagatgaga gccaaacgga agaccctagc     900

acctccccat ccccaggtac ccgagccacc agccagccac ctaattcctc agaggctggc     960

agaaaaccct gggaacggag caactcggtg gagaaacctg tgtcctcgtt gctgtccaga    1020

accccgtctg tggcaaagag ccccgaagct aagagccccc ttcagtcgca gcctcactct    1080

agggtgaagc ctgctgggag tgtgaatgac gtgggcctgg atgccttaga tttggaccgg    1140

atgaaacagg agatcctgga ggaggtggtt cgggagctgc acaaggtgaa ggaggagatc    1200

attgatgcca tcaggtag                                                  1218


<210>  8
<211>  405
<212>  PRT
<213>  Mus musculus

<400>  8

Met Ser Glu Gln Ser Ile Cys Gln Ala Arg Ala Ser Val Met Val Tyr

1               5                   10                  15

Asp Asp Thr Ser Lys Lys Trp Val Pro Ile Lys Pro Gly Gln Gln Gly
            20                  25                  30

Phe Ser Arg Ile Asn Ile Tyr His Asn Thr Ala Ser Ser Thr Phe Arg
        35                  40                  45

Val Val Gly Val Lys Leu Gln Asp Gln Gln Val Val Ile Asn Tyr Ser
    50                  55                  60

Ile Val Lys Gly Leu Lys Tyr Asn Gln Ala Thr Pro Thr Phe His Gln
65                  70                  75                  80

Trp Arg Asp Ala Arg Gln Val Tyr Gly Leu Asn Phe Ala Ser Lys Glu
                85                  90                  95

Glu Ala Thr Thr Phe Ser Asn Ala Met Leu Phe Ala Leu Asn Ile Met
            100                 105                 110

Asn Ser Gln Glu Gly Gly Pro Ser Thr Gln Arg Gln Val Gln Asn Gly
        115                 120                 125

Pro Ser Pro Glu Glu Met Asp Ile Gln Arg Arg Gln Val Met Glu Gln
    130                 135                 140

Gln His Arg Gln Glu Ser Leu Glu Arg Arg Ile Ser Ala Thr Gly Pro
145                 150                 155                 160

Ile Leu Pro Pro Gly His Pro Ser Ser Ala Ala Ser Thr Thr Leu Ser
                165                 170                 175

Cys Ser Gly Pro Pro Pro Pro Pro Pro Pro Pro Val Pro Pro Pro Pro
            180                 185                 190

Thr Gly Ser Thr Pro Pro Pro Pro Pro Pro Leu Pro Ala Gly Gly Ala
        195                 200                 205

Gln Gly Thr Asn His Asp Glu Ser Ser Ala Ser Gly Leu Ala Ala Ala
    210                 215                 220

Leu Ala Gly Ala Lys Leu Arg Arg Val Gln Arg Pro Glu Asp Ala Ser
225                 230                 235                 240

Gly Gly Ser Ser Pro Ser Gly Thr Ser Lys Ser Asp Ala Asn Arg Ala
                245                 250                 255

Ser Ser Gly Gly Gly Gly Gly Gly Leu Met Glu Glu Met Asn Lys Leu
            260                 265                 270

Leu Ala Lys Arg Arg Lys Ala Ala Ser Gln Thr Asp Lys Pro Ala Asp
        275                 280                 285

Arg Lys Glu Asp Glu Ser Gln Thr Glu Asp Pro Ser Thr Ser Pro Ser
    290                 295                 300

Pro Gly Thr Arg Ala Thr Ser Gln Pro Pro Asn Ser Ser Glu Ala Gly
305                 310                 315                 320

Arg Lys Pro Trp Glu Arg Ser Asn Ser Val Glu Lys Pro Val Ser Ser
                325                 330                 335

Leu Leu Ser Arg Thr Pro Ser Val Ala Lys Ser Pro Glu Ala Lys Ser
            340                 345                 350

Pro Leu Gln Ser Gln Pro His Ser Arg Val Lys Pro Ala Gly Ser Val
        355                 360                 365

Asn Asp Val Gly Leu Asp Ala Leu Asp Leu Asp Arg Met Lys Gln Glu
    370                 375                 380

Ile Leu Glu Glu Val Val Arg Glu Leu His Lys Val Lys Glu Glu Ile
385                 390                 395                 400

Ile Asp Ala Ile Arg
                405

<210>  9
<211>  1182
<212>  DNA
<213>  Rattus norvegicus

<400> 9
atgagcgaac agagtatctg ccaagcacgg gcctccgtga tggtctacga tgacaccagt 60

aagaaatggg taccaatcaa gcctggccag cagggattca gccggatcaa catctaccac 120

aacactgcca gcaacacttt cagggttgta ggggtcaagc tacaggatca gcaggttgtg 180

atcaattatt caattgtgaa agggctgaag tacaatcagg caacacccac cttccatcag 240

tggcgagacg cccgtcaggt ctatggctta aactttgcga gtaagggaga agcaaccaca 300

ttctccaacg cgatgctctt tgccctgaac atcatgaact cccaagaagg aggcccctcc 360

acacagcgtc aggtgcagaa tggcccctct cctgaggaga tggacatcca gagaagacaa 420

gtaatggagc agcagcaccg ccaggagtct ctggagagaa gaatctccgc cacagggccc 480

attctccccc ctgggcatcc gtcatcggca gccagcgcca ccttctcctg tagtggacct 540

ccacctccac ctccacctcc agttccacct ccacccacag ggtctactcc cccgcccccg 600

cccccgctgc ctgctggagg agcccagggg accaaccacg atgagagctc tgcatcagga 660

ctggctgctg ctctggcagg agccaagcta aggagggtgc agcggccaga ggatgcatct 720

ggaggctcca gtcctagcgg gacttcaaag tccgatgcca accgggcaag cagtggggga 780

ggaggaggag gcctcatgga agaaatgaac aagctgctgg ctaagaggag aaaggcagcc 840

tcccagacag acaagcccgc tgacagaaag gaagatgaga accaaacgga agatcctagc 900

acctccccat ccccagggag ccgagccacc agccagccac ctaattcctc agaggctggc 960

cgaaagccct gggaacggag caactcggtg gagaaacctg tgtcctcgtt gctgtccagg 1020

gtgaagcctg ctgggagtgt gaatgacgtg ggcctggatg ccttagattt ggaccggatg 1080

aaacaggaga ttctggagga ggtggtccga gagctccaca aggtgaagga ggagatcata 1140

gatgccatca ggcaggaact aagtgggatc agcaccacat aa 1182

<210> 10
<211> 393
<212> PRT
<213> Rattus norvegicus

<400> 10

Met Ser Glu Gln Ser Ile Cys Gln Ala Arg Ala Ser Val Met Val Tyr
1               5                   10                  15

Asp Asp Thr Ser Lys Lys Trp Val Pro Ile Lys Pro Gly Gln Gln Gly

            20                  25                  30

Phe Ser Arg Ile Asn Ile Tyr His Asn Thr Ala Ser Asn Thr Phe Arg
        35                  40                  45

Val Val Gly Val Lys Leu Gln Asp Gln Gln Val Val Ile Asn Tyr Ser
    50                  55                  60

Ile Val Lys Gly Leu Lys Tyr Asn Gln Ala Thr Pro Thr Phe His Gln
65                  70                  75                  80

Trp Arg Asp Ala Arg Gln Val Tyr Gly Leu Asn Phe Ala Ser Lys Gly
                85                  90                  95

Glu Ala Thr Thr Phe Ser Asn Ala Met Leu Phe Ala Leu Asn Ile Met
            100                 105                 110

Asn Ser Gln Glu Gly Gly Pro Ser Thr Gln Arg Gln Val Gln Asn Gly
        115                 120                 125

Pro Ser Pro Glu Glu Met Asp Ile Gln Arg Arg Gln Val Met Glu Gln
    130                 135                 140

Gln His Arg Gln Glu Ser Leu Glu Arg Arg Ile Ser Ala Thr Gly Pro
145                 150                 155                 160

Ile Leu Pro Pro Gly His Pro Ser Ser Ala Ala Ser Ala Thr Phe Ser
                165                 170                 175

Cys Ser Gly Pro Pro Pro Pro Pro Pro Pro Pro Val Pro Pro Pro Pro
            180                 185                 190

Thr Gly Ser Thr Pro Pro Pro Pro Pro Pro Leu Pro Ala Gly Gly Ala
        195                 200                 205

Gln Gly Thr Asn His Asp Glu Ser Ser Ala Ser Gly Leu Ala Ala Ala
    210                 215                 220

Leu Ala Gly Ala Lys Leu Arg Arg Val Gln Arg Pro Glu Asp Ala Ser
225                 230                 235                 240

Gly Gly Ser Ser Pro Ser Gly Thr Ser Lys Ser Asp Ala Asn Arg Ala
                245                 250                 255

Ser Ser Gly Gly Gly Gly Gly Gly Leu Met Glu Glu Met Asn Lys Leu
            260                 265                 270

Leu Ala Lys Arg Arg Lys Ala Ala Ser Gln Thr Asp Lys Pro Ala Asp
        275                 280                 285

Arg Lys Glu Asp Glu Asn Gln Thr Glu Asp Pro Ser Thr Ser Pro Ser
    290                 295                 300

Pro Gly Ser Arg Ala Thr Ser Gln Pro Pro Asn Ser Ser Glu Ala Gly
305                 310                 315                 320

Arg Lys Pro Trp Glu Arg Ser Asn Ser Val Glu Lys Pro Val Ser Ser
                325                 330                 335

Leu Leu Ser Arg Val Lys Pro Ala Gly Ser Val Asn Asp Val Gly Leu
            340                 345                 350

Asp Ala Leu Asp Leu Asp Arg Met Lys Gln Glu Ile Leu Glu Glu Val
        355                 360                 365

Val Arg Glu Leu His Lys Val Lys Glu Glu Ile Ile Asp Ala Ile Arg
    370                 375                 380

Gln Glu Leu Ser Gly Ile Ser Thr Thr
385                 390

<210>  11
<211>  1257
<212>  DNA
<213>  Gallus gallus

<400>  11
atggcgggca ttgaacagag tatttgccaa gcccgggctt cagttatggt ctatgacgat      60

accagtaaga aatgggtgcc aatcaaacct ggacagcagg gattcagcag aatcaacata     120

tatcacaaca cggccacaaa caccttcagg gttgttggag ttaaactgca agatcaacaa     180

gtagtgatta attactcaat tgtgaaagga ctgaagtaca atcaagcaac acctaccttt     240

catcaatggc gcgatgcacg gcaagtctat ggcttgaatt ttgcaagcaa agaagaggct 300

actacgttct ccaatgcaat gctgtttgct ctgaatataa tgaattcaca agatggaggt 360

ccagctgccc agcgccaggt ccagaatggg ccatctccag atgagatgga agcacaaagg 420

agacaagtga tggagcagca gcaacagcgc caagaatctc tggaaagaag aacttctacc 480

acaggcccag ctctcccacc cggccatccc agcggtgctt cagtgatccc tgcttcatcc 540

gggggccccc cacctccgcc accccccccg gcccctccgc cccccatggg agccgctccc 600

ccaccaccac ccccgctgcc agccggctcc ggccaagggg ctgccagtga agatgggtcg 660

gtgtcagggc tcgcggctgc tctggctggt gccaaactga ggagagttca gcggccagaa 720

gatggttcag gagggtccag ccccagcggg gtctctaaga gcgatgccaa tcgaacaagt 780

agtggaggag gcagcggagg actaatggaa gaaatgaata aattactggc aaaaaggagg 840

aaagcagcgt cgcagtcaga caagccaggt gacaaaaagg aagaggaaag ccaaaatgat 900

gatgctagca cctctccttc aaccagtaca cggggaccca cccagcagca gcaaaattca 960

tcagactctg ggaagaagcc atgggaaagg agcaattctg ttgaaaagcc tgtatcttca 1020

ttactgtcta gaaatccatc catggtgaag agctgtgaag ctaagagccc cacacaatcc 1080

cacgtgtctt ctaggatgaa gccagtaagc agcagcaatg atgtggctat ggatgcctta 1140

gattttgatc ggatgaaaca ggaaatattg gaggaagttg taagagagtt acacaaagtg 1200

aaagaggaga taattgatgc catacggcag gagttgagta ggatcagtac aacatga 1257

<210> 12
<211> 418
<212> PRT
<213> Gallus gallus

<400> 12

Met Ala Gly Ile Glu Gln Ser Ile Cys Gln Ala Arg Ala Ser Val Met
1               5                   10                  15

Val Tyr Asp Asp Thr Ser Lys Lys Trp Val Pro Ile Lys Pro Gly Gln
            20                  25                  30

Gln Gly Phe Ser Arg Ile Asn Ile Tyr His Asn Thr Ala Thr Asn Thr
        35                  40                  45

Phe Arg Val Val Gly Val Lys Leu Gln Asp Gln Gln Val Val Ile Asn
    50                  55                  60

Tyr Ser Ile Val Lys Gly Leu Lys Tyr Asn Gln Ala Thr Pro Thr Phe
65                  70                  75                  80

His Gln Trp Arg Asp Ala Arg Gln Val Tyr Gly Leu Asn Phe Ala Ser
                85                  90                  95

Lys Glu Glu Ala Thr Thr Phe Ser Asn Ala Met Leu Phe Ala Leu Asn
            100                 105                 110

Ile Met Asn Ser Gln Asp Gly Gly Pro Ala Ala Gln Arg Gln Val Gln
        115                 120                 125

Asn Gly Pro Ser Pro Asp Glu Met Glu Ala Gln Arg Arg Gln Val Met
    130                 135                 140

Glu Gln Gln Gln Gln Arg Gln Glu Ser Leu Glu Arg Arg Thr Ser Thr
145                 150                 155                 160

Thr Gly Pro Ala Leu Pro Pro Gly His Pro Ser Gly Ala Ser Val Ile
                165                 170                 175

Pro Ala Ser Ser Gly Gly Pro Pro Pro Pro Pro Pro Pro Pro Ala Pro
            180                 185                 190

Pro Pro Pro Met Gly Ala Ala Pro Pro Pro Pro Pro Pro Leu Pro Ala
        195                 200                 205

Gly Ser Gly Gln Gly Ala Ala Ser Glu Asp Gly Ser Val Ser Gly Leu
    210                 215                 220

Ala Ala Ala Leu Ala Gly Ala Lys Leu Arg Arg Val Gln Arg Pro Glu
225                 230                 235                 240

Asp Gly Ser Gly Gly Ser Ser Pro Ser Gly Val Ser Lys Ser Asp Ala
                245                 250                 255

Asn Arg Thr Ser Ser Gly Gly Gly Ser Gly Gly Leu Met Glu Glu Met

260                 265                 270

Asn Lys Leu Leu Ala Lys Arg Arg Lys Ala Ala Ser Gln Ser Asp Lys
        275                 280                 285

Pro Gly Asp Lys Lys Glu Glu Glu Ser Gln Asn Asp Asp Ala Ser Thr
    290                 295                 300

Ser Pro Ser Thr Ser Thr Arg Gly Pro Thr Gln Gln Gln Gln Asn Ser
305                 310                 315                 320

Ser Asp Ser Gly Lys Lys Pro Trp Glu Arg Ser Asn Ser Val Glu Lys
                325                 330                 335

Pro Val Ser Ser Leu Leu Ser Arg Asn Pro Ser Met Val Lys Ser Cys
            340                 345                 350

Glu Ala Lys Ser Pro Thr Gln Ser His Val Ser Ser Arg Met Lys Pro
        355                 360                 365

Val Ser Ser Ser Asn Asp Val Ala Met Asp Ala Leu Asp Phe Asp Arg
    370                 375                 380

Met Lys Gln Glu Ile Leu Glu Glu Val Val Arg Glu Leu His Lys Val
385                 390                 395                 400

Lys Glu Glu Ile Ile Asp Ala Ile Arg Gln Glu Leu Ser Arg Ile Ser
                405                 410                 415

Thr Thr

<210>  13
<211>  1053
<212>  DNA
<213>  Homo sapiens

<400>  13
atgggtagca agaaactaaa acgagtgggt ttatcacaag agctgtgtga ccgtctgagt      60

agacatcaga tccttacctg tcaggacttt ttatgtcttt ccccactgga gcttatgaag     120

gtgactggtc tgagttatcg aggtgtccat gaacttctat gtatggtcag cagggcctgt     180

gccccaaaga tgcaaacggc ttatgggata aaagcacaaa ggtctgctga tttctcacca 240

gcattcttat ctactaccct ttctgctttg gacgaagccc tgcatggtgg tgtggcttgt 300

ggatccctca cagagattac aggtccacca ggttgtggaa aaactcagtt ttgtataatg 360

atgagcattt tggctacatt acccaccaac atgggaggat tagaaggagc tgtggtgtac 420

attgacacag agtctgcatt tagtgctgaa agactggttg aaatagcaga atcccgtttt 480

cccagatatt ttaacactga agaaaagtta cttttgacaa gtagtaaagt tcatctttat 540

cgggaactca cctgtgatga agttctacaa aggattgaat ctttggaaga agaaattatc 600

tcaaaaggaa ttaaacttgt gattcttgac tctgttgctt ctgtggtcag aaaggagttt 660

gatgcacaac ttcaaggcaa tctcaaagaa agaaacaagt tcttggcaag agaggcatcc 720

tccttgaagt atttggctga ggagttttca atcccagtta tcttgacgaa tcagattaca 780

acccatctga gtggagccct ggcttctcag gcagacctgg tgtctccagc tgatgatttg 840

tccctgtctg aaggcacttc tggatccagc tgtgtgatag ccgcactagg aaatacctgg 900

agtcacagtg tgaatacccg gctgatcctc cagtaccttg attcagagag aagacagatt 960

cttattgcca agtcccctct ggctcccttc acctcatttg tctacaccat caaggaggaa 1020

ggcctggttc ttcaagccta tggaaattcc tag 1053

<210> 14
<211> 350
<212> PRT
<213> Homo sapiens

<400> 14

Met Gly Ser Lys Lys Leu Lys Arg Val Gly Leu Ser Gln Glu Leu Cys
1               5                   10                  15

Asp Arg Leu Ser Arg His Gln Ile Leu Thr Cys Gln Asp Phe Leu Cys
            20                  25                  30

Leu Ser Pro Leu Glu Leu Met Lys Val Thr Gly Leu Ser Tyr Arg Gly
        35                  40                  45

Val His Glu Leu Leu Cys Met Val Ser Arg Ala Cys Ala Pro Lys Met
    50                  55                  60

Gln Thr Ala Tyr Gly Ile Lys Ala Gln Arg Ser Ala Asp Phe Ser Pro
65                  70                  75                  80

Ala Phe Leu Ser Thr Thr Leu Ser Ala Leu Asp Glu Ala Leu His Gly
                85                  90                  95

Gly Val Ala Cys Gly Ser Leu Thr Glu Ile Thr Gly Pro Pro Gly Cys
            100                 105                 110

Gly Lys Thr Gln Phe Cys Ile Met Met Ser Ile Leu Ala Thr Leu Pro
        115                 120                 125

Thr Asn Met Gly Gly Leu Glu Gly Ala Val Val Tyr Ile Asp Thr Glu
    130                 135                 140

Ser Ala Phe Ser Ala Glu Arg Leu Val Glu Ile Ala Glu Ser Arg Phe
145                 150                 155                 160

Pro Arg Tyr Phe Asn Thr Glu Glu Lys Leu Leu Leu Thr Ser Ser Lys
                165                 170                 175

Val His Leu Tyr Arg Glu Leu Thr Cys Asp Glu Val Leu Gln Arg Ile
            180                 185                 190

Glu Ser Leu Glu Glu Glu Ile Ile Ser Lys Gly Ile Lys Leu Val Ile
        195                 200                 205

Leu Asp Ser Val Ala Ser Val Val Arg Lys Glu Phe Asp Ala Gln Leu
    210                 215                 220

Gln Gly Asn Leu Lys Glu Arg Asn Lys Phe Leu Ala Arg Glu Ala Ser
225                 230                 235                 240

Ser Leu Lys Tyr Leu Ala Glu Glu Phe Ser Ile Pro Val Ile Leu Thr
                245                 250                 255

Asn Gln Ile Thr Thr His Leu Ser Gly Ala Leu Ala Ser Gln Ala Asp
            260                 265                 270

Leu Val Ser Pro Ala Asp Asp Leu Ser Leu Ser Glu Gly Thr Ser Gly

275                 280                 285

Ser Ser Cys Val Ile Ala Ala Leu Gly Asn Thr Trp Ser His Ser Val
    290                 295                 300

Asn Thr Arg Leu Ile Leu Gln Tyr Leu Asp Ser Glu Arg Arg Gln Ile
305                 310                 315                 320

Leu Ile Ala Lys Ser Pro Leu Ala Pro Phe Thr Ser Phe Val Tyr Thr
                325                 330                 335

Ile Lys Glu Glu Gly Leu Val Leu Gln Ala Tyr Gly Asn Ser
            340                 345                 350

<210>  15
<211>  1053
<212>  DNA
<213>  Mus musculus

<400>  15
atgagcagca agaaactaag acgagtgggt ttatctccag agctgtgtga ccgtttaagc      60

agataccaga ttgttaactg tcagcacttt ttaagtctct ccccactaga acttatgaaa     120

gtgactggcc tgagttacag aggtgtccac gagcttcttc atacagtaag caaggcctgt     180

gccccgcaga tgcaaacggc ttatgagtta aagacacgaa ggtctgcaca tctctcaccg     240

gcattcctgt ctactaccct gtgcgccttg gatgaagcat tgcacggtgg tgtgccttgt     300

ggatctctca cagagattac aggtccacca ggttgcggaa aaactcagtt ttgcataatg     360

atgagtgtct tagctacatt acctaccagc ctgggaggat tagaaggggc tgtggtctac     420

atcgacacag agtctgcatt tactgctgag agactggttg agattgcgga atctcgtttt     480

ccacaatatt ttaacactga ggaaaaattg cttctgacca gcagtagagt tcatctttgc     540

cgagagctca cctgtgaggg gcttctacaa aggcttgagt ctttggagga agagatcatt     600

tcgaaaggag ttaagcttgt gattgttgac tccattgctt ctgtggtcag aaaggagttt     660

gacccgaagc ttcaaggcaa catcaaagaa aggaacaagt tcttgggcaa aggagcgtcc     720

ttactgaagt acctggcagg ggagttttca atcccagtta tcttgacgaa tcaaattacg     780

acccatctga gtggagccct cccttctcaa gcagacctgg tgtctccagc tgatgatttg     840

tccctgtctg aaggcacttc tggatccagc tgtttggtag ctgcactagg aaacacatgg     900

ggtcactgtg tgaacacccg gctgattctc cagtaccttg attcagagag aaggcagatt 960

ctcattgcca agtctcctct ggctgccttc acctcctttg tctacaccat caagggggaa 1020

ggcctggttc ttcaaggcca cgaaagacca tag 1053

<210> 16
<211> 350
<212> PRT
<213> Mus musculus

<400> 16

Met Ser Ser Lys Lys Leu Arg Arg Val Gly Leu Ser Pro Glu Leu Cys
1               5                   10                  15

Asp Arg Leu Ser Arg Tyr Gln Ile Val Asn Cys Gln His Phe Leu Ser
            20                  25                  30

Leu Ser Pro Leu Glu Leu Met Lys Val Thr Gly Leu Ser Tyr Arg Gly
        35                  40                  45

Val His Glu Leu Leu His Thr Val Ser Lys Ala Cys Ala Pro Gln Met
    50                  55                  60

Gln Thr Ala Tyr Glu Leu Lys Thr Arg Arg Ser Ala His Leu Ser Pro
65                  70                  75                  80

Ala Phe Leu Ser Thr Thr Leu Cys Ala Leu Asp Glu Ala Leu His Gly
                85                  90                  95

Gly Val Pro Cys Gly Ser Leu Thr Glu Ile Thr Gly Pro Pro Gly Cys
            100                 105                 110

Gly Lys Thr Gln Phe Cys Ile Met Met Ser Val Leu Ala Thr Leu Pro
        115                 120                 125

Thr Ser Leu Gly Gly Leu Glu Gly Ala Val Val Tyr Ile Asp Thr Glu
    130                 135                 140

Ser Ala Phe Thr Ala Glu Arg Leu Val Glu Ile Ala Glu Ser Arg Phe
145                 150                 155                 160

Pro Gln Tyr Phe Asn Thr Glu Glu Lys Leu Leu Leu Thr Ser Ser Arg
                165                 170                 175

Val His Leu Cys Arg Glu Leu Thr Cys Glu Gly Leu Leu Gln Arg Leu
            180                 185                 190

Glu Ser Leu Glu Glu Glu Ile Ile Ser Lys Gly Val Lys Leu Val Ile
        195                 200                 205

Val Asp Ser Ile Ala Ser Val Val Arg Lys Glu Phe Asp Pro Lys Leu
    210                 215                 220

Gln Gly Asn Ile Lys Glu Arg Asn Lys Phe Leu Gly Lys Gly Ala Ser
225                 230                 235                 240

Leu Leu Lys Tyr Leu Ala Gly Glu Phe Ser Ile Pro Val Ile Leu Thr
                245                 250                 255

Asn Gln Ile Thr Thr His Leu Ser Gly Ala Leu Pro Ser Gln Ala Asp
            260                 265                 270

Leu Val Ser Pro Ala Asp Asp Leu Ser Leu Ser Glu Gly Thr Ser Gly
        275                 280                 285

Ser Ser Cys Leu Val Ala Ala Leu Gly Asn Thr Trp Gly His Cys Val
    290                 295                 300

Asn Thr Arg Leu Ile Leu Gln Tyr Leu Asp Ser Glu Arg Arg Gln Ile
305                 310                 315                 320

Leu Ile Ala Lys Ser Pro Leu Ala Ala Phe Thr Ser Phe Val Tyr Thr
                325                 330                 335

Ile Lys Gly Glu Gly Leu Val Leu Gln Gly His Glu Arg Pro
            340                 345                 350

<210>  17
<211>  1017
<212>  DNA
<213>  Arabidopsis thaliana

<400>  17
atgacggaat ttgaactaat ggagctgtta gatgttggaa tgaaagagat aagatcagca      60

atttcattca tcagtgaagc tacttctcca ccatgtcaat ctgctcgatc tttactggag     120

aagaaggtcg aaaacgaaca tttatcaggt catcttccta cacatttgaa ggggttagat     180

tataccttgt gtggtgggat accttttggt gttcttactg agttagttgg tcctcctggt     240

attggtaaat cacagttttg catgaaactt gcgttatcag cttcgtttcc agtagcttat     300

ggaggattag atggtcgtgt gatatacata gatgtggaat ccaagtttag ttcaagaagg     360

gtgatagaga tgggactgga aagctttccg gaagtgtttc atcttaaagg aatggcacaa     420

gagatggctg gaagaatcct tgttttgcgt ccaacatctt tagctaactt tactgaaagt     480

atacaagaac tcaagaattc aattcttcaa aaccaagtaa agcttctagt gattgatagt     540

atgacagctc ttctttcagg cgaaaacaaa ccaggagctc agagacaacc tcagttgggt     600

tggcatatct ctttcttaaa atcgcttgct gaattttcac ggattcctat agtggtgact     660

aatcaagtta gatctcaaaa ccgcgatgaa actagtcagt attctttcca agctaaagtt     720

aaagatgaat tcaaagacaa cacaaagaca tatgattctc accttgttgc tgcattgggg     780

attaactggg ctcatgctgt aaccatccga ctggtccttg aagccaagtc aggtcagaga     840

atcattaagg tggcaaaatc tcctatgtcg cctcctttag ccttcccgtt ccatataaca     900

tcagctggga tttcattgct gagcgacaac gggactgaac tgaaaggtcc aggaatcaac     960

accattcatg ctcgagggca cagcgacatg ataaattttc acggggactg ctcgtag      1017

<210>  18
<211>  338
<212>  PRT
<213>  Arabidopsis thaliana

<400>  18

Met Thr Glu Phe Glu Leu Met Glu Leu Leu Asp Val Gly Met Lys Glu
1               5                   10                  15

Ile Arg Ser Ala Ile Ser Phe Ile Ser Glu Ala Thr Ser Pro Pro Cys
            20                  25                  30

Gln Ser Ala Arg Ser Leu Leu Glu Lys Lys Val Glu Asn Glu His Leu
        35                  40                  45

Ser Gly His Leu Pro Thr His Leu Lys Gly Leu Asp Tyr Thr Leu Cys
    50                  55                  60

Gly Gly Ile Pro Phe Gly Val Leu Thr Glu Leu Val Gly Pro Pro Gly
65                  70                  75                  80

Ile Gly Lys Ser Gln Phe Cys Met Lys Leu Ala Leu Ser Ala Ser Phe
                85                  90                  95

Pro Val Ala Tyr Gly Gly Leu Asp Gly Arg Val Ile Tyr Ile Asp Val
            100                 105                 110

Glu Ser Lys Phe Ser Ser Arg Arg Val Ile Glu Met Gly Leu Glu Ser
        115                 120                 125

Phe Pro Glu Val Phe His Leu Lys Gly Met Ala Gln Glu Met Ala Gly
    130                 135                 140

Arg Ile Leu Val Leu Arg Pro Thr Ser Leu Ala Asn Phe Thr Glu Ser
145                 150                 155                 160

Ile Gln Glu Leu Lys Asn Ser Ile Leu Gln Asn Gln Val Lys Leu Leu
                165                 170                 175

Val Ile Asp Ser Met Thr Ala Leu Leu Ser Gly Glu Asn Lys Pro Gly
            180                 185                 190

Ala Gln Arg Gln Pro Gln Leu Gly Trp His Ile Ser Phe Leu Lys Ser
        195                 200                 205

Leu Ala Glu Phe Ser Arg Ile Pro Ile Val Val Thr Asn Gln Val Arg
    210                 215                 220

Ser Gln Asn Arg Asp Glu Thr Ser Gln Tyr Ser Phe Gln Ala Lys Val
225                 230                 235                 240

Lys Asp Glu Phe Lys Asp Asn Thr Lys Thr Tyr Asp Ser His Leu Val
                245                 250                 255

Ala Ala Leu Gly Ile Asn Trp Ala His Ala Val Thr Ile Arg Leu Val

260                 265                 270

Leu Glu Ala Lys Ser Gly Gln Arg Ile Ile Lys Val Ala Lys Ser Pro
        275                 280                 285

Met Ser Pro Pro Leu Ala Phe Pro Phe His Ile Thr Ser Ala Gly Ile
    290                 295                 300

Ser Leu Leu Ser Asp Asn Gly Thr Glu Leu Lys Gly Pro Gly Ile Asn
305                 310                 315                 320

Thr Ile His Ala Arg Gly His Ser Asp Met Ile Asn Phe His Gly Asp
                325                 330                 335

Cys Ser

<210>  19
<211>  594
<212>  DNA
<213>  Homo sapiens

<400>  19
atgtcaggac tggccgctgc catagctggg gccaagctga gaagagtcca acggccagaa      60

gacgcatctg gaggctccag tcccagtggg acctcaaagt ccgatgccaa ccgggcaagc     120

agcgggggtg gcggaggagg cctcatggag gaaatgaaca aactgctggc caagaggaga     180

aaagcagcct cccagtcaga caagccagcc gagaagaagg aagatgaaag ccaaatggaa     240

gatcctagta cctccccctc tccggggacc cgagcagcca gccagccacc taactcctca     300

gaggctggcc ggaagccctg ggagcggagc aactcggtgg agaagcctgt gtcctcgatt     360

ctgtccagaa ccccgtctgt ggcaaagagc cccgaagcta agagccccct tcagtcgcag     420

cctcactcta ggatgaagcc tgctgggagc gtgaatgaca tggccctgga tgccttcgac     480

ttggaccgga tgaagcagga gatcctagag gaggtggtga gagagctcca caaggtgaag     540

gaggagatca tcgacgccat caggcaggag ctgagtggga tcagcaccac gtaa           594

<210>  20
<211>  197
<212>  PRT
<213>  Homo sapiens

<400>  20

Met Ser Gly Leu Ala Ala Ala Ile Ala Gly Ala Lys Leu Arg Arg Val
1               5                   10                  15

Gln Arg Pro Glu Asp Ala Ser Gly Gly Ser Ser Pro Ser Gly Thr Ser
            20                  25                  30

Lys Ser Asp Ala Asn Arg Ala Ser Ser Gly Gly Gly Gly Gly Gly Leu
        35                  40                  45

Met Glu Glu Met Asn Lys Leu Leu Ala Lys Arg Arg Lys Ala Ala Ser
    50                  55                  60

Gln Ser Asp Lys Pro Ala Glu Lys Lys Glu Asp Glu Ser Gln Met Glu
65                  70                  75                  80

Asp Pro Ser Thr Ser Pro Ser Pro Gly Thr Arg Ala Ala Ser Gln Pro
                85                  90                  95

Pro Asn Ser Ser Glu Ala Gly Arg Lys Pro Trp Glu Arg Ser Asn Ser
            100                 105                 110

Val Glu Lys Pro Val Ser Ser Ile Leu Ser Arg Thr Pro Ser Val Ala
        115                 120                 125

Lys Ser Pro Glu Ala Lys Ser Pro Leu Gln Ser Gln Pro His Ser Arg
    130                 135                 140

Met Lys Pro Ala Gly Ser Val Asn Asp Met Ala Leu Asp Ala Phe Asp
145                 150                 155                 160

Leu Asp Arg Met Lys Gln Glu Ile Leu Glu Glu Val Val Arg Glu Leu
                165                 170                 175

His Lys Val Lys Glu Glu Ile Ile Asp Ala Ile Arg Gln Glu Leu Ser
            180                 185                 190

Gly Ile Ser Thr Thr
        195

## Description

### Technical Field

The present invention relates to a composition for cleaving and/or binding single-stranded DNA.

### Background Art

Recombinant DNA technology, which makes full use of genetic engineering techniques, is an important technology that is essential for the development of the current biotechnology industry. For example, in recent years, in the field of medicine, it is almost impossible to supply pharmaceutical products to meet demand in the development and production of protein preparations whose efficacy has been focused, without using such recombinant DNA technology. Thus, it may be no exaggeration to say that the development of the current medical field cannot be anticipated without this technology. In addition, it is desired to further develop DNA manipulation technology, not only for the development of protein preparations but also for the development of protein reagents used in research and development.

The most basic technique for dealing with DNA recombination operations includes the cleavage of DNA and the binding of a free terminus. A majority of DNA molecules used as targets of such operations have been double-stranded DNA molecules. To date, since DNA has been often used as a "tool for encoding a protein and expressing it," most researchers have been interested in the progress of a technique of manipulating a double-stranded DNA. However, at present, with the expansion of a biotechnology target region, opportunities for manipulating a single-stranded DNA have been increased. For example, when a DNA chip or the like is produced, it is essential to prepare a single-stranded DNA having a desired sequence and a desired length, and further, an enzyme and the like used to manipulate such single-stranded DNA are also considered as important factors. A large number of methods for cleaving a single-stranded DNA have been reported so far. However, as methods for binding a single-stranded DNA to another single-stranded DNA, there have been reported only several methods such as a method using T4 RNA ligase (Patent Document 1 and Non-Patent Document 1), a method using thermostable ligase derived from archaebacteria (Patent Document 2), and a method using thermostable ligase derived from thermophilic phage TS2126 (Patent Document 3).
Under such circumstances, it is desired to develop an enzyme or a composition capable of manipulating available single-stranded DNA molecules.

[Patent Document 1] JP Patent Publication (Kokai) No. 2002-171983 A (the entire text)
[Patent Document 2] JP Patent Publication (Kokai) No. 6-62847 A (1994) (the entire text)
[Patent Document 3] US Patent No. 6,818,425 (the entire text)
[Non-Patent Document 1] Nishigaki et al., Mol Divers 4: 187-90, 1998

### Disclosure of the Invention

### Problems to be Solved by the Invention

Under the aforementioned circumstances, the present inventors have conducted intensive studies. As a result, the inventors have found that an Evl protein has activity of cleaving a single-stranded DNA and binding the 5'-terminus of such single-stranded DNA to the 3'-terminus thereof, thereby completing the present invention.
Accordingly, it is an object of the present invention to provide a composition for cleaving and/or binding a single-stranded DNA.

### Means for Solving the Problems

Specifically, the present invention relates to the following (1) to (12):
(1) A first aspect of the present invention relates to "a composition for cleaving a single-stranded DNA and/or binding the 5'-terminus of such single-stranded DNA to the 3'-terminus thereof, which comprises an Evl protein described in the following (a) or (b):
   (a) a protein having the amino acid sequence shown in SEQ ID NO: 2, 4, 6, 8, 10, 12, or 20; or
   (b) a polypeptide, which has an amino acid sequence comprising a substitution, deletion, or insertion of one or several amino acids with respect to the amino acid sequence shown in SEQ ID NO: 2, 4, 6, 8, 10, 12, or 20, and which has activity of cleaving a single-stranded DNA and binding the 5'-terminus of such single-stranded DNA to the 3'-terminus thereof."
(2) A second aspect of the present invention relates to "the composition according to (1) above, wherein it further comprises Mg²⁺ or Ca²⁺."
(3) A third aspect of the present invention relates to "the composition according to (2) above, wherein the concentration of the Mg²⁺ is between 0.5 mM and 2.0 mM."
(4) A fourth aspect of the present invention relates to "the composition according to (3) above, wherein it further comprises a Rad51B protein and ATP."
(5) A fifth aspect of the present invention relates to "the composition according to (4) above, wherein the molar ratio of the Evl protein to the Rad51B is from 1 : 0.5 to 1 : 4."
(6) A sixth aspect of the present invention relates to "the composition according to (4) or (5) above, wherein the concentration of the ATP is between 0.5 mM and 2.0 mM."
(7) A seventh aspect of the present invention relates to "the composition according to any one of (1) to (3) above, wherein it further comprises a DNA topoisomerase type I protein."
(8) An eighth aspect of the present invention relates to "a composition for cleaving a single-stranded DNA and/or binding the 5'-terminus of such single-stranded DNA to the 3'-terminus thereof, which comprises a recombinant vector comprising a nucleic acid described in the following (a) or (b):
   (a) a nucleic acid having the nucleotide sequence shown in SEQ ID NO: 1, 3, 5, 7, 9, 11, or 19; or
   (b) a nucleic acid, which hybridizes under stringent conditions with the complementary strand of the nucleic acid having the nucleotide sequence shown in SEQ ID NO: 1, 3, 5, 7, 9, 11, or 19, and which encodes a polypeptide having activity of cleaving a single-stranded DNA and binding the 5'-terminus of such single-stranded DNA to the 3'-terminus thereof."
(9) A ninth aspect of the present invention relates to "a composition for cleaving a single-stranded DNA and/or binding the 5'-terminus of such single-stranded DNA to the 3'-terminus thereof, which comprises a recombinant vector comprising a nucleic acid described in the following (a) or (b):
   (a) a nucleic acid encoding a polypeptide having the amino acid sequence shown in SEQ ID NO: 2, 4, 6, 8, 10, 12, or 20; or
   (b) a nucleic acid encoding a polypeptide, which has an amino acid sequence comprising a substitution, deletion, or insertion of one or several amino acids with respect to the amino acid sequence shown in SEQ ID NO: 2, 4, 6, 8, 10, 12, or 20, and which has activity of cleaving a single-stranded DNA and binding the 5'-terminus of such single-stranded DNA to the 3'-terminus thereof."
(10) A tenth aspect of the present invention relates to "a method for producing a single-stranded DNA marker by reacting the composition according to any one of (1) to (9) above with a single-stranded DNA."
(11) An eleventh aspect of the present invention relates to "a single-stranded DNA marker produced by the method according to (10) above."
(12) A twelfth aspect of the present invention relates to "a composition for inhibiting Evl protein activity, which comprises one or multiple compounds selected from the compound group consisting of aclarubicin, dequalinium, DIDS, β-rubromycin, and 3-ATA."

### Effects of the Invention

According to the method of the present invention, a single-stranded DNA can be cleaved, and/or the 5'-terminus of such single-stranded DNA can be bound to the 3'-terminus thereof.

### Brief Description of the Drawings

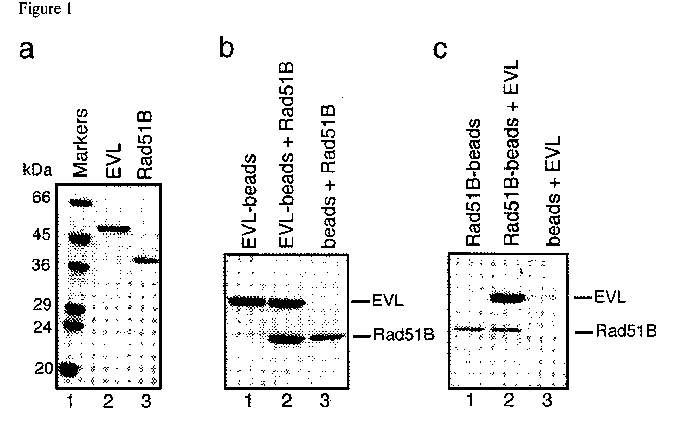

Figure 1 shows the results indicating that an Evl protein binds to a Rad51B protein. In Figure 1a, the purified Evl protein (0.5 µg) and Rad51B protein (0.5 µg) were electrophoresed by 12% SDS-PAGE, and were then strained with Coomassie Brilliant Blue. Lane 1 indicates markers, and lanes 2 and 3 are the purified Evl and the purified Rad51B, respectively. Figure 1b shows the results indicating the interaction of Evl-Rad51B. Lanes 1 and 2 indicate the results of a binding experiment using Evl-bound beads in the absence (lane 1) or presence (lane 2) of Rad51B. Lane 3 indicates the experimental results of a negative control, in which Affi-Gel beads to which no protein had bound were used. Figure 1c shows the results of the interaction of Evl-Rad51B. Lanes 1 and 2 indicate the results of a binding experiment using Rad51B-bound beads in the absence (lane 1) or presence (lane 2) of Evl. Lane 3 indicates the experimental results of a negative control, in which Affi-Gel beads to which no protein had bound were used.

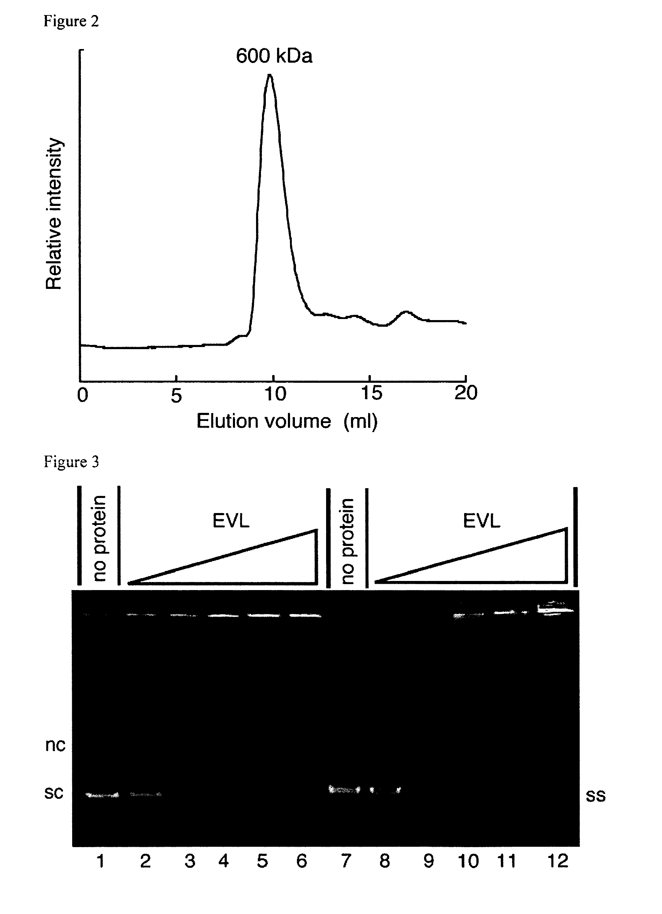

Figure 2 shows the results obtained by gel filtration of the purified Evl protein. The Evl protein was eluted at a position of molecular weight of approximately 600 kDa.
Figure 3 shows the results obtained by examining the DNA binding ability of the Evl protein. Lanes 1-6 and lanes 7-12 indicate the results obtained by examining the ability of the Evl protein to bind to a double-stranded DNA and to a single-stranded DNA, respectively. Lanes 1 and 7 indicate the results of a negative control, to which no Evl protein was added. The concentrations of Evl proteins used in such binding experiments are 0.2 µM (lanes 2 and 8), 0.4 µM (lanes 3 and 9), 0.8 µM (lanes 4 and 10), 1.6 µM (lanes 5 and 11), and 3.2 µM (lanes 6 and 12). The symbol "nc" indicates a nicked circular double-stranded DNA, the symbol "sc" indicates a supercoiled circular double-stranded DNA, and the symbol "ss" indicates a single-stranded DNA (this also applies to other figures).

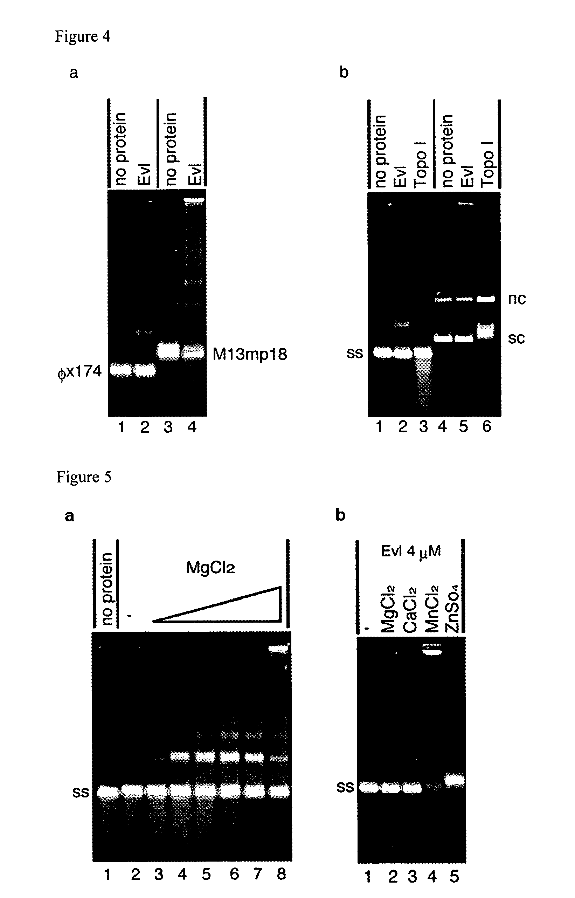

Figure 4 shows the results obtained by examining the activity of cleaving a single-stranded DNA and binding the 5'-terminus of such single-stranded DNA to the 3'-terminus thereof. Figure 4a shows the results obtained by examining the activity of Evl to a circular single-stranded DNA. Lanes 1 and 2 indicate the results obtained using a φ×174 circular single-stranded DNA. Lanes 3 and 4 indicate the results obtained using a M13mp18 circular single-stranded DNA. In addition, lanes 1 and 3 indicate the results of control experiments in which no Evl protein was used, and lanes 2 and 4 indicate the results of experiments in which the Evl protein was used. Figure 4b shows the results obtained by examining the activity of Evl to double-stranded, supercoiled DNA and nicked DNA. A φ×174 circular single-stranded DNA (20 µM, lanes 1-3), or φ×174 supercoiled and nicked double-stranded DNA molecules (10 µM, lanes 4-6) were used to examine the influence of Evl (4 µM, lanes 2 and 5) and Escherichia coli-derived topoisomerase type I (5 units, lanes 3 and 6). Lanes 1 and 4 indicate the results of control experiments in which no Evl protein was used.
Figure 5 shows the influence of Mg²⁺ and divalent metal ions on the activity of an Evl protein. Figure 5a shows the results obtained by examining the influence of Mg²⁺ on the activity of the Evl protein. A catenation reaction was carried out on a φ×174 circular single-stranded DNA (20 µM) using an Evl protein (4 µM) in the presence of various concentrations of Mg²⁺. Lane 1 indicates the results of control experiments in which no Evl protein was used. In addition, lanes 2-8 indicate experimental results in the presence of Mg²⁺ in concentrations of 0 mM, 0.5 mM, 1.0 mM, 1.25 mM, 1.5 mM, 1.75 mM, and 2.0 mM, respectively. Figure 5b shows the results obtained by examining the influence of divalent metal ions on the activity of an Evl protein. A catenation reaction was carried out on a φ×174 circular single-stranded DNA (20 µM) using an Evl protein (4 µM) in the presence of various types of divalent metal ions. Lane 1 indicates the results of a control experiment in which no metal ion was used. In addition, lanes 2-5 indicate experimental results in the presence of 1 mM metal ions of several types as shown in the figure.

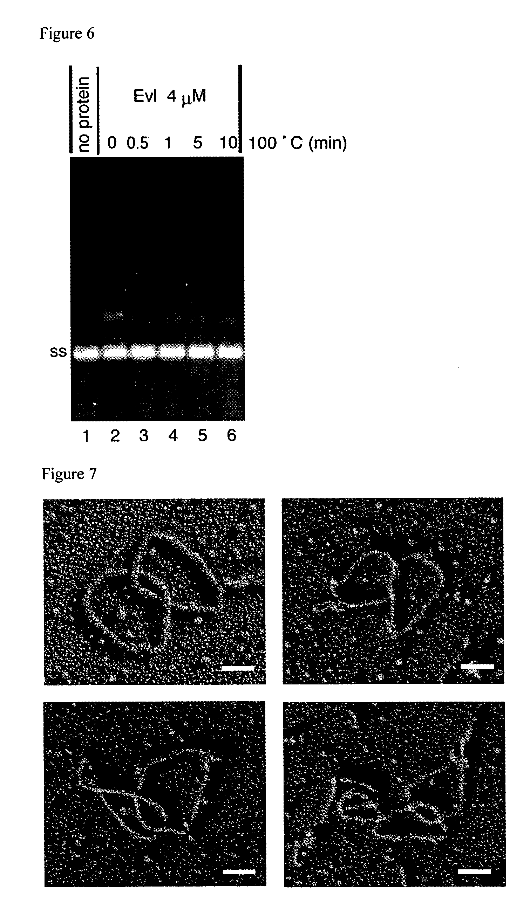

Figure 6 shows the results obtained by examining the heat stability of a reaction product obtained using an Evl protein. The lanes show the results obtained by incubating at 100°C a reaction product obtained by reacting a φ×174 circular single-stranded DNA (20 µM) with an Evl protein (4 µM) for 0 minute (lane 2), 0.5 minutes (lane 3), 1 minute (lane 4), 5 minutes (lane 5), and 10 minutes (lane 6), followed by agarose gel electrophoresis. Lane 1 indicates the results of a control experiment in which no Evl protein was used.
Figure 7 shows the results obtained by observing a reaction product obtained using an Evl protein under an electron microscope. The scale bar indicates 100 nm.

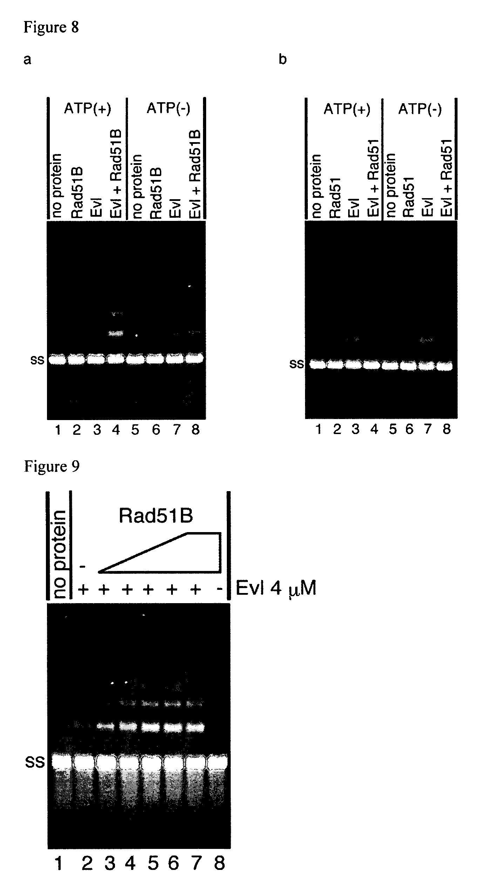

Figure 8 shows the results indicating that a Rad51B protein promotes the activity of an Evl protein. Figure 8a shows the results obtained by examining the influence of the Rad51B protein on the activity of the Evl protein. Lanes 1 and 5 indicate the results of control experiments in which neither the Evl nor the Rad51B protein was used. Lanes 2 and 6 indicate the results of experiments that were carried out using a 4 µM Rad51B protein in the absence of the Evl protein. Lanes 3 and 7 indicate the results of experiments that were carried out using a 4 µM Evl protein in the absence of the Rad51B protein. Lanes 4 and 8 indicate the results of experiments that were carried out using a 4 µM Evl protein and a 4 µM Rad51B protein. Further, lanes 1-4 indicate the results of experiments that were carried out in the presence of ATP, and lanes 5-8 indicate the results of experiments that were carried out in the absence of ATP. Figure 8b shows the results obtained by examining the influence of a Rad51 protein on the activity of an Evl protein. Lanes 1 and 5 indicate the results of control experiments in which neither the Evl nor the Rad51 protein was used. Lanes 2 and 6 indicate the results of experiments that were carried out using a 4 µM Rad51 protein in the absence of the Evl protein. Lanes 3 and 7 indicate the results of experiments that were carried out using a 4 µM Evl protein in the absence of the Rad51 protein. Lanes 4 and 8 indicate the results of experiments that were carried out using a 4 µM Evl protein and a 4 µM Rad51 protein. Further, lanes 1-4 indicate the results of experiments that were carried out in the presence of ATP, and lanes 5-8 indicate the results of experiments that were carried out in the absence of ATP.
Figure 9 shows the results obtained by examining the concentration-dependent effect of a Rad51B protein to promote the catenation activity of an Evl protein. The Rad51B protein was mixed in a concentration of 0.5 to 8 µM with a 4 µM Evl protein, and the activity was then measured. Lane 1 indicates the results of a control experiment in which neither the Rad51B protein nor the Evl protein was added, and lane 8 indicates the results of a control experiment in which a 8 µM Rad51B protein was added and no Evl protein was added. Lanes 2-7 indicate the results of experiments in which the Rad51B protein was added in concentrations of 0 µM, 0.5 µM, 1.0 µM, 2.0 µM, 4.0 µM, and 8.0 µM, respectively, in the presence of 4 µM Evl.

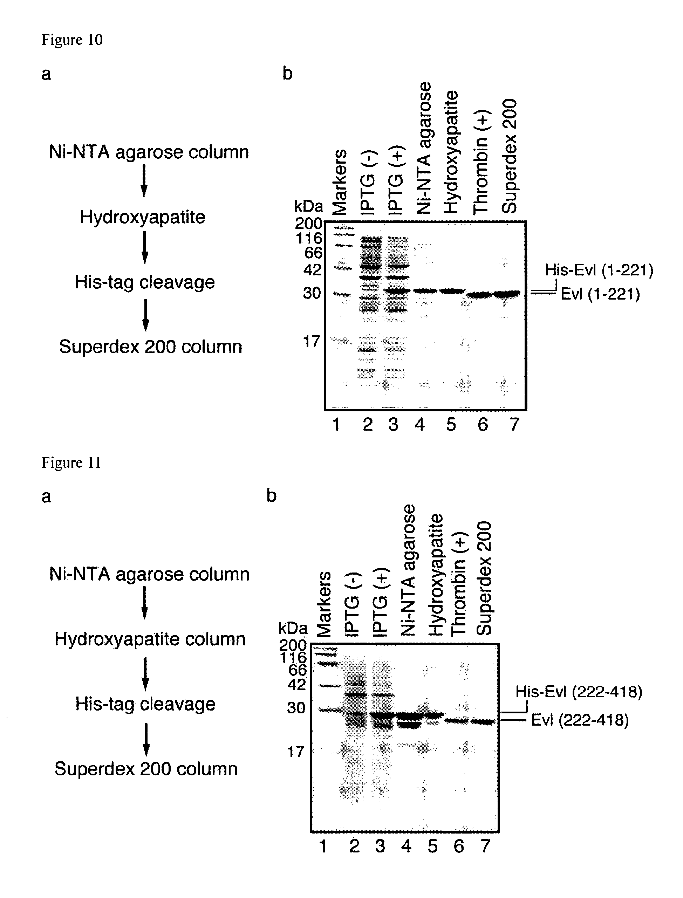

Figure 10 shows the results of purification of an Evl (1-221) mutant. Figure 10a shows a process of purifying the Evl (1-221) mutant. Figure 10b shows the results obtained by subjecting the sample in each purification step to 15% SDS-PAGE and then staining the resultant sample with Coomassie Brilliant Blue. Lane 1 indicates a molecular weight marker. Lanes 2 and 3 indicate a whole host cell extract before and after addition of IPTG, respectively. Lanes 4-7 indicate an Ni-NTA agarose fraction, a hydroxyapatite pass-through fraction, a fraction after a thrombin treatment, and a Superdex200 peak fraction, respectively.
Figure 11 shows the results of purification of an Evl (222-418) mutant. Figure 11a shows a process of purifying the Evl (222-418) mutant. Figure 11b shows the results obtained by subjecting the sample in each purification step to 15% SDS-PAGE and then staining the resultant sample with Coomassie Brilliant Blue. Lane 1 indicates a molecular weight marker. Lanes 2 and 3 indicate a whole host cell extract before and after addition of IPTG, respectively. Lanes 4-7 indicate an Ni-NTA agarose fraction, a hydroxyapatite peak fraction, a fraction after a thrombin treatment, and a Superdex200 peak fraction, respectively.

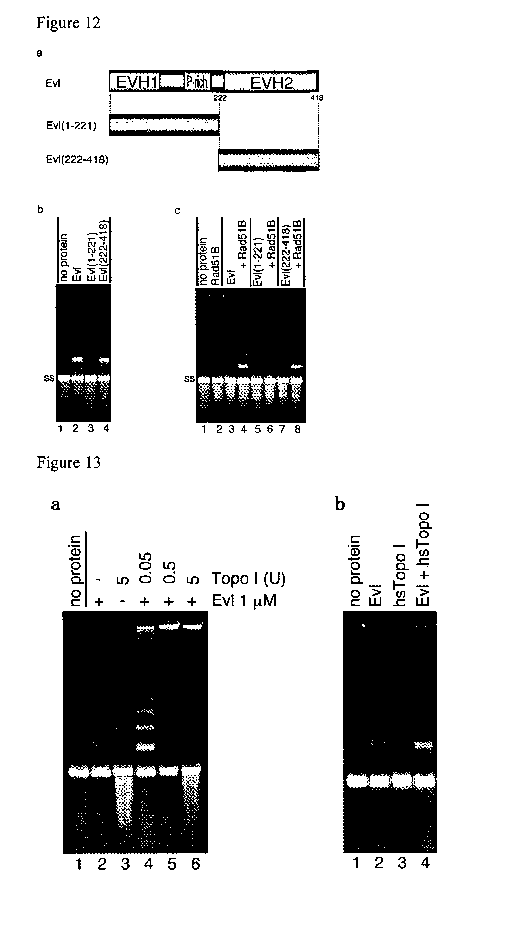

Figure 12 shows the results obtained by examining the activity of the EVH2 domain of an Evl protein. Figure 12a is a schematic view showing the domain structure of the Evl protein and the deletion mutants used in the present example. EVH1, a proline-rich region, and EVH2 are shown in the figure. Figure 12b shows the results obtained by analyzing the activities of Evl deletion mutants. Lanes 1-4 indicate the results obtained by examining such activity in the absence of protein, in the presence of the Evl protein (4 µM), in the presence of the Evl (1-221) mutant (4 µM), and in the presence of the Evl (222-418) mutant (4 µM), respectively. Figure 12c shows the results obtained by analyzing the influence of a Rad51B protein on the activities of Evl deletion mutants. Lane 1 indicates the results of an experiment in which no protein was used. Lane 2 indicates the results of an experiment in which only the Rad51B protein was added. Lanes 3 and 4, lanes 5 and 6, and lanes 7 and 8 indicate the results of experiments in which the Evl protein (4 µM), the Evl (1-221) mutant (4 µM), and the Evl (222-418) mutant (4 µM) were added, respectively. Moreover, lanes 4, 6, and 8 indicate the results of experiments in which the Rad51B protein (4 µM) was further added.
Figure 13 shows the results regarding the effect of the coexistence of a DNA topoisomerase type I protein and an Evl protein to promote the catenation of a single-stranded DNA. Figure 13a shows the effect of TopoI (derived from Escherichia coli) to promote the catenation of a single-stranded DNA. Lane 1 indicates the results of a negative control in which only a single-stranded DNA was reacted. Lane 2 indicates the results obtained by reacting a 1 µM Evl protein with a single-stranded DNA. Lane 3 indicates the results obtained by reacting 5 U TopoI (E. coli, New England Biolabs) with a single-stranded DNA. Lane 4 indicates the results obtained by reacting a single-stranded DNA in the coexistence of a 1 µM Evl protein and 0.05 U TopoI. Lane 5 indicates the results obtained by reacting a single-stranded DNA in the coexistence of a 1 µM Evl protein and 0.5 U TopoI. Lane 6 indicates the results obtained by reacting a single-stranded DNA in the coexistence of a 1 µM Evl protein and 5 U TopoI. Figure 13b shows the effect of hsTopoI (derived from a human) to promote the catenation of a single-stranded DNA. Lane 1 indicates the results of a negative control in which only a single-stranded DNA was reacted. Lane 2 indicates the results obtained by reacting a 0.5 µM Evl protein with a single-stranded DNA. Lane 3 indicates the results obtained by reacting 2.8 nM hsTopoI (human, Jena Bioscience) with a single-stranded DNA. Lane 4 indicates the results obtained by reacting a single-stranded DNA in the coexistence of a 0.5 µM Evl protein and 2.8 nM hsTopoI.

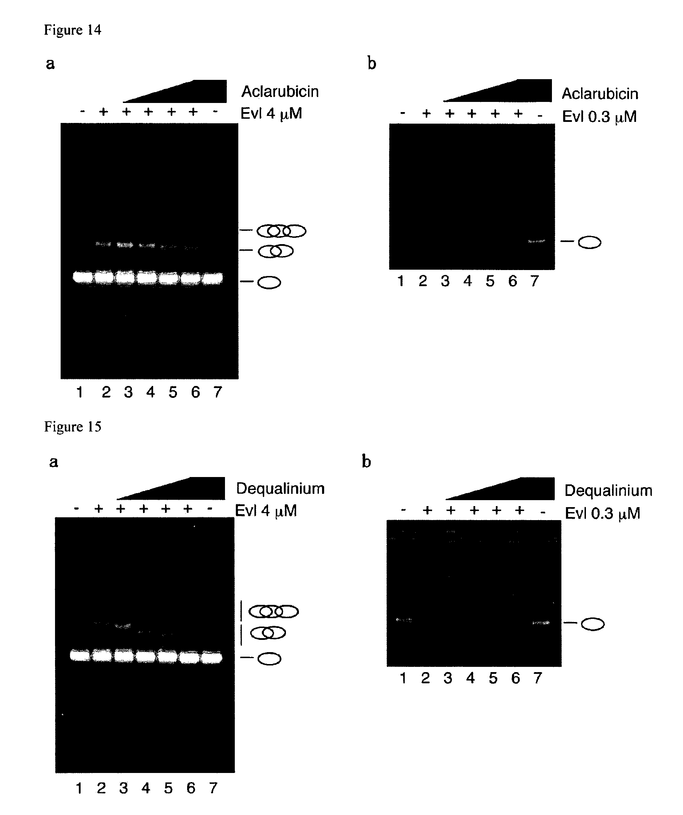

Figure 14 shows the influence of aclarubicin on the activity of an Evl protein to catenate a single-stranded DNA. Figure 14a shows the effect of aclarubicin to inhibit the single-stranded DNA catenation activity of the Evl protein. Lane 1 indicates the results of a negative control in which only a single-stranded DNA was reacted. Lane 2 indicates the results obtained by reacting a 4 µM Evl protein with a single-stranded DNA. Lane 3 indicates the results obtained by reacting a 4 µM Evl protein and 1 µM aclarubicin with a single-stranded DNA. Lane 4 indicates the results obtained by reacting a 4 µM Evl protein and 5 µM aclarubicin with a single-stranded DNA. Lane 5 indicates the results obtained by reacting a 4 µM Evl protein and 10 µM aclarubicin with a single-stranded DNA. Lane 6 indicates the results obtained by reacting a 4 µM Evl protein and 20 µM aclarubicin with a single-stranded DNA. Lane 7 indicates the results obtained by reacting 20 µM aclarubicin with a single-stranded DNA. Figure 14b shows the influence of aclarubicin on the DNA binding activity of an Evl protein. The experiments were carried out in the same manner as that of Figure 14a with the exception that the concentration of the reacted Evl protein was set at 0.3 µM.
Figure 15 shows the influence of dequalinium on the activity of an Evl protein to catenate a single-stranded DNA. Figure 15a shows the effect of dequalinium to inhibit the single-stranded DNA catenation activity of the Evl protein. Lane 1 indicates the results of a negative control in which only a single-stranded DNA was reacted. Lane 2 indicates the results obtained by reacting a 4 µM Evl protein with a single-stranded DNA. Lane 3 indicates the results obtained by reacting a 4 µM Evl protein and 1 µM dequalinium with a single-stranded DNA. Lane 4 indicates the results obtained by reacting a 4 µM Evl protein and 5 µM dequalinium with a single-stranded DNA. Lane 5 indicates the results obtained by reacting a 4 µM Evl protein and 10 µM dequalinium with a single-stranded DNA. Lane 6 indicates the results obtained by reacting a 4 µM Evl protein and 20 µM dequalinium with a single-stranded DNA. Lane 7 indicates the results obtained by reacting 20 µM dequalinium with a single-stranded DNA. Figure 15b shows the influence of dequalinium on the DNA binding activity of an Evl protein. The experiments were carried out in the same manner as that of Figure 15a with the exception that the concentration of the reacted Evl protein was set at 0.3 µM.

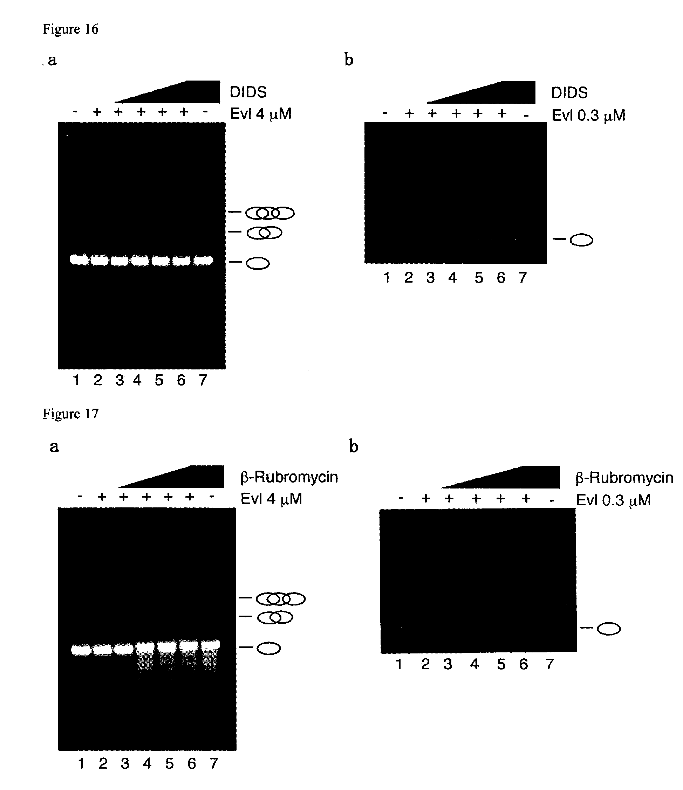

Figure 16 shows the influence of DIDS on the activity of an Evl protein to catenate a single-stranded DNA. Figure 16a shows the effect of DIDS to inhibit the single-stranded DNA catenation activity of the Evl protein. Lane 1 indicates the results of a negative control in which only a single-stranded DNA was reacted. Lane 2 indicates the results obtained by reacting a 4 µM Evl protein with a single-stranded DNA. Lane 3 indicates the results obtained by reacting a 4 µM Evl protein and 1 µM DIDS with a single-stranded DNA. Lane 4 indicates the results obtained by reacting a 4 µM Evl protein and 5 µM DIDS with a single-stranded DNA. Lane 5 indicates the results obtained by reacting a 4 µM Evl protein and 10 µM DIDS with a single-stranded DNA. Lane 6 indicates the results obtained by reacting a 4 µM Evl protein and 20 µM DIDS with a single-stranded DNA. Lane 7 indicates the results obtained by reacting 20 µM DIDS with a single-stranded DNA. Figure 16b shows the influence of DIDS on the DNA binding activity of an Evl protein. The experiments were carried out in the same manner as that of Figure 16a with the exception that the concentration of the reacted Evl protein was set at 0.3 µM.
Figure 17 shows the influence of β-rubromycin on the activity of an Evl protein to catenate a single-stranded DNA. Figure 17a shows the effect of β-rubromycin to inhibit the single-stranded DNA catenation activity of the Evl protein. Lane 1 indicates the results of a negative control in which only a single-stranded DNA was reacted. Lane 2 indicates the results obtained by reacting a 4 µM Evl protein with a single-stranded DNA. Lane 3 indicates the results obtained by reacting a 4 µM Evl protein and 1 µM β-rubromycin with a single-stranded DNA. Lane 4 indicates the results obtained by reacting a 4 µM Evl protein and 5 µM β-rubromycin with a single-stranded DNA. Lane 5 indicates the results obtained by reacting a 4 µM Evl protein and 10 µM β-rubromycin with a single-stranded DNA. Lane 6 indicates the results obtained by reacting a 4 µM Evl protein and 20 µM β-rubromycin with a single-stranded DNA. Lane 7 indicates the results obtained by reacting 20 µM β-rubromycin with a single-stranded DNA. Figure 17b shows the influence of β-rubromycin on the DNA binding activity of an Evl protein. The experiments were carried out in the same manner as that of Figure 17a with the exception that the concentration of the reacted Evl protein was set at 0.3 µM.

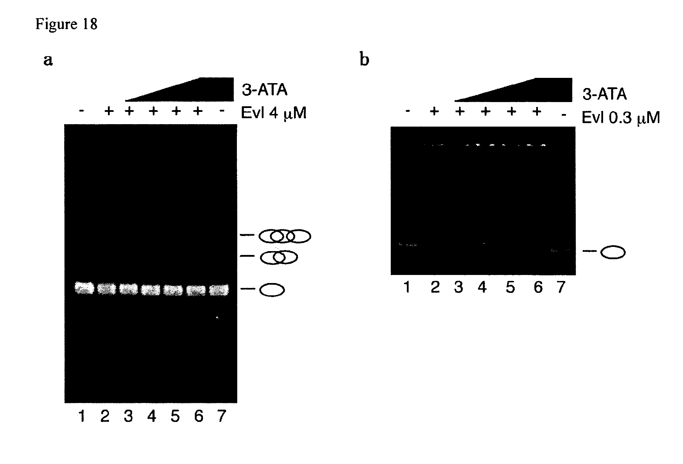

Figure 18 shows the influence of 3-ATA on the activity of an Evl protein to catenate a single-stranded DNA. Figure 18a shows the effect of 3-ATA to inhibit the single-stranded DNA catenation activity of the Evl protein. Lane 1 indicates the results of a negative control in which only a single-stranded DNA was reacted. Lane 2 indicates the results obtained by reacting a 4 µM Evl protein with a single-stranded DNA. Lane 3 indicates the results obtained by reacting a 4 µM Evl protein and 1 µM 3-ATA with a single-stranded DNA. Lane 4 indicates the results obtained by reacting a 4 µM Evl protein and 5 µM 3-ATA with a single-stranded DNA. Lane 5 indicates the results obtained by reacting a 4 µM Evl protein and 10 µM 3-ATA with a single-stranded DNA. Lane 6 indicates the results obtained by reacting a 4 µM Evl protein and 20 µM 3-ATA with a single-stranded DNA. Lane 7 indicates the results obtained by reacting 20 µM 3-ATA with a single-stranded DNA. Figure 18b shows the influence of 3-ATA on the DNA binding activity of an Evl protein. The experiments were carried out in the same manner as that of Figure 18a with the exception that the concentration of the reacted Evl protein was set at 0.3 µM.

### Best Mode for Carrying Out the Invention

A first embodiment of the present invention relates to "a composition for cleaving a single-stranded DNA and/or binding the 5'-terminus of such single-stranded DNA to the 3'-terminus thereof, which comprises an Evl protein described in the following (a) or (b):
(a) a protein having the amino acid sequence shown in SEQ ID NO: 2, 4, 6, 8, 10, 12, or 20; or
(b) a polypeptide, which has an amino acid sequence comprising a substitution, deletion, or insertion of one or several amino acids with respect to the amino acid sequence shown in SEQ ID NO: 2, 4, 6, 8, 10, 12, or 20, and which has activity of cleaving a single-stranded DNA and binding the 5'-terminus of such single-stranded DNA to the 3'-terminus thereof."
The term "Evl protein" is used herein to mean a protein having an amino acid sequence identical to or substantially identical to the amino acid sequence shown in SEQ ID NO: 2, 4, 6, 8, 10, or 12. The description "a protein having an amino acid sequence ... substantially identical to ..." is used herein to mean a protein, which has an amino acid sequence showing amino acid identity of approximately 60% or more, preferably approximately 70% or more, more preferably approximately 80%, 81%, 82%, 83%, 84%, 85%, 86%, 87%, 88%, 89%, 90%, 91%, 92%, 93%, 94%, 95%, 96%, 97% or 98%, and most preferably approximately 99%, with the amino acid sequence shown in SEQ ID NO: 2, 4, 6, 8, 10, or 12, and which has activity of cleaving a single-stranded DNA and binding the 5'-terminus of such single-stranded DNA to the 3'-terminus thereof.
It is to be noted that the terms "polypeptide" and "protein" have the same meanings in the present specification, unless otherwise specified.

Alternatively, the protein having an amino acid sequence substantially identical to the amino acid sequence shown in SEQ ID NO: 2, 4, 6, 8, 10, or 12 may be a protein, which has an amino acid sequence comprising a deletion, substitution, or addition of one or several amino acids (preferably approximately 1 to 30, more preferably approximately 1 to 10, and further preferably 1 to 5 amino acids) with respect to the amino acid sequence shown in SEQ ID NO: 2, 4, 6, 8, 10, or 12, and which has activity of cleaving a single-stranded DNA and binding the 5'-terminus of such single-stranded DNA to the 3'-terminus thereof.
The aforementioned deletion, addition and substitution of amino acids may be present in an isolated, native polypeptide. Otherwise, a gene encoding the protein of the present invention may be modified by a method known in the present technical field, so that such deletion, addition or substitution of amino acids may be newly introduced into a protein. For example, substitution of specific amino acid residue(s) may be carried out by substituting nucleotides with other nucleotides according to a known method such as a Gupped duplex method or a Kunkel method, or a method equivalent thereto, using a commercially available kit (for example, MutanTM-G (TAKARA), MutanTM-K (TAKARA), etc.).

The C-terminus of the protein used in the present invention is generally a carboxyl group (-COOH) or carboxylate (-COO-). However, such carboxyl group may be chemically modified with an amide (-CONH₂), an ester (-COOR), or the like. Herein, R in such ester includes C₁₋₆ alkyl groups (for example, methyl, ethyl, n-propyl, isopropyl, and n-butyl), C₃₋₈ cycloalkyl groups (for example, cyclopentyl and cyclohexyl), C₁₋₆ aryl groups (for example, phenyl and α-naphthyl), phenyl-C₁₋₂ alkyl groups (for example, benzyl and phenethyl), α-naphthyl-C₁₋₂ alkyl groups (for example, α-naphthylmethyl), and other groups. Otherwise, there may also be used a pivaloyloxymethyl ester, which has been generally known as an ester for oral use. When the Evl protein of the present invention has a carboxyl group not only at the C-terminus thereof but also in the polypeptide chain thereof, an amidated or esterified carboxyl group is also included in the protein of the present invention. Examples of such ester include the above-described esters. Likewise, the N-terminus of the protein of the present invention is generally an amino group (-NH₂). However, such amino group may be chemically modified with a C₁₋₆ acyl group such as a formyl group or an acetyl group. Moreover, the protein of the present invention also includes: a protein in which a glutamyl group generated as a result of the cleavage of the N-terminal side *in vivo* is converted to pyroglutamic acid; a protein in which a substituent on the side chain of an intramolecular amino acid (for example, -OH, -SH, an amino group, an imidazole group, an indole group, a guanidino group, etc.) is chemically modified with a suitable functional group (for example, formyl, acetyl, etc.); and a sugar chain-binding protein.

A peptide having a partial amino acid sequence contained in the Evl protein of the present invention (which is also referred to as a "partial peptide") may also be included in the composition of the present invention, as long as the partial peptide has activity of cleaving a single-stranded DNA and binding the 5'-terminus of such single-stranded DNA to the 3'-terminus thereof. An example of such partial peptide is a polypeptide having an amino acid sequence identical to or substantially identical to the amino acid sequence shown in SEQ ID NO: 20. The description "a polypeptide having an amino acid sequence ... substantially identical to ..." is used herein to mean a polypeptide, which has an amino acid sequence showing amino acid identity of approximately 60% or more, preferably approximately 70% or more, more preferably approximately 80%, 81%, 82%, 83%, 84%, 85%, 86%, 87%, 88%, 89%, 90%, 91%, 92%, 93%, 94%, 95%, 96%, 97% or 98%, and most preferably approximately 99%, with the amino acid sequence shown in SEQ ID NO: 20, and which has activity of cleaving a single-stranded DNA and binding the 5'-terminus of such single-stranded DNA to the 3'-terminus thereof.

Alternatively, the polypeptide having an amino acid sequence substantially identical to the amino acid sequence shown in SEQ ID NO: 20 may be a polypeptide, which has an amino acid sequence comprising a deletion, substitution, or addition of one or several amino acids (preferably approximately 1 to 30, more preferably approximately 1 to 10, and further preferably 1 to 5 amino acids) with respect to the amino acid sequence shown in SEQ ID NO: 20, and which has activity of cleaving a single-stranded DNA and binding the 5'-terminus of such single-stranded DNA to the 3'-terminus thereof.
The aforementioned deletion, addition and substitution of amino acids may be present in an isolated, native polypeptide. Otherwise, a gene encoding the protein of the present invention may be modified by a method known in the present technical field, so that such deletion, addition or substitution of amino acids may be newly introduced into a protein. For example, substitution of specific amino acid residue(s) may be carried out by substituting nucleotides with other nucleotides according to a known method such as a Gupped duplex method or a Kunkel method, or a method equivalent thereto, using a commercially available kit (for example, MutanTM-G (TAKARA), MutanTM-K (TAKARA), etc.).

The Evl protein or a partial peptide thereof may be obtained from a natural source, or it may be obtained in the form of a recombinant. In order to obtain such recombinant, a recombinant vector is necessary for expression of the Evl protein or a partial peptide thereof. Moreover, such recombinant vector may be contained in the composition of the present invention. A nucleic acid encoding the Evl protein or a partial peptide thereof is inserted into the recombinant vector, such that the nucleic acid can be expressed therein. Herein, the description "can be expressed" is used to mean a state in which a nucleic acid encoding an Evl protein having a desired amino acid sequence or a partial peptide thereof, with a proper reading frame, is inserted into an expression vector, such that the Evl protein or a partial peptide thereof can be expressed therein, and in which other necessary components such as a promoter and a selective marker are also constructed in the vector, such that they can properly function.
The term "a nucleic acid encoding the Evl protein" is used herein to include, not only a nucleic acid having the nucleotide sequence shown in SEQ ID NO: 1, 3, 5, 7, 9, or 11, but also a nucleic acid, which hybridizes under stringent conditions with a nucleic acid having a nucleotide sequence complementary to the nucleotide sequence shown in SEQ ID NO: 1, 3, 5, 7, 9, or 11, and which encodes a polypeptide having activity of cleaving a single-stranded DNA and binding the 5'-terminus of such single-stranded DNA to the 3'-terminus thereof.
An example of DNA capable of hybridizing under stringent conditions with the nucleic acid having the nucleotide sequence shown in SEQ ID NO: 1, 3, 5, 7, 9, or 11 is a nucleic acid having a nucleotide sequence showing polynucleotide sequence homology of preferably approximately 70% or more, more preferably approximately 80%, 81%, 82%, 83%, 84%, 85%, 86%, 87%, 88%, 89%, 90%, 91%, 92%, 93%, 94%, 95%, 96%, 97% or 98%, and most preferably approximately 99%, with the nucleotide sequence shown in SEQ ID NO: 1, 3, 5, 7, 9, or 11.

The description "a nucleic acid encoding a partial peptide of the Evl protein" is used herein to include, not only a nucleic acid having the nucleotide sequence shown in SEQ ID NO: 19, but also a nucleic acid, which hybridizes under stringent conditions with a nucleic acid having a nucleotide sequence complementary to the nucleotide sequence shown in SEQ ID NO: 19 and which encodes a polypeptide having activity of cleaving a single-stranded DNA and binding the 5'-terminus of such single-stranded DNA to the 3'-terminus thereof.
An example of DNA capable of hybridizing under stringent conditions with the nucleic acid having the nucleotide sequence shown in SEQ ID NO: 19 is a nucleic acid having a nucleotide sequence showing polynucleotide sequence homology of preferably approximately 70% or more, more preferably approximately 80%, 81%, 82%, 83%, 84%, 85%, 86%, 87%, 88%, 89%, 90%, 91%, 92%, 93%, 94%, 95%, 96%, 97% or 98%, and most preferably approximately 99%, with the nucleotide sequence shown in SEQ ID NO: 19.

The term "stringent conditions" is used herein to mean conditions for hybridization, which are easily determined by persons skilled in the art. Such stringent conditions are various empirical conditions that are generally dependent on the length of a probe, a washing temperature, and a salt concentration. In general, as a probe becomes longer, a temperature necessary for appropriate annealing also increases. On the other hand, as a probe becomes shorter, such temperature decreases. Hybrid formation generally depends on the ability of a nucleic acid in which a complementary strand is allowed to reanneal under temperature conditions slightly lower than the melting point thereof.
Specific examples of low stringent conditions include washing a filter at a temperature between 37°C and 42°C in a 0.1 × SSC and 0.1% SDS solution in the step of washing the filter after completion of the hybridization. Specific examples of high stringent conditions include washing a filter at 65°C in a 5 × SSC and 0.1% SDS solution in such washing step. By further increasing such stringent conditions, a highly homologous polynucleotide can be obtained.

A nucleic acid encoding an Evl protein or a partial peptide thereof can be obtained from the cells of eukaryotes including mammals such as a human, a rat, a mouse, and a sheep, according to a common method. Alternatively, it is also possible to obtain the nucleic acid sequence information of the Evl protein, which has already been known, from database and the like, and to obtain an Evl gene derived from a desired organism species based on the sequence information. Such Evl gene can be cloned based on common knowledge in the present technical field. The gene can be obtained by preparing a cDNA library from cells that express the gene and then applying a common screening method. Alternatively, the gene may also be obtained by preparing RNA from cells that express the gene, synthesizing cDNA from the RNA using reverse transcriptase, then preparing PCR primers based on the gene sequence, and then amplifying the cDNA using the prepared PCR primers.

A recombinant vector, into which a nucleic acid encoding an Evl protein or a partial peptide thereof has been incorporated, can be obtained by ligating the nucleic acid to a suitable vector. When a recombinant vector is subjected to a cloning procedure, the type of the recombinant vector is not particularly limited, as long as it is able to replicate in a host. Moreover, as a vector used for expression of the Evl protein or a partial peptide thereof, a vector that is able to replicate in a host and allows a DNA fragment encoding the protein to express therein, such as promoter, can be used.

Examples of an available vector include plasmid DNA and phage DNA. Examples of such plasmid DNA include Escherichia coli-derived plasmids (for example, pBR322, pBR325, pUC118, pUC 119, pUC18, pUC19, pCBD-C, pET15b, etc.), Bacillus subtilis-derived plasmids (for example, pUB110, pTP5, pC194, etc.), and yeast-derived plasmids (for example, YEp13, YEp24, YCp50, YIp30, etc.). An example of phage DNA is λ phage. Moreover, animal virus vectors such as retrovirus or vaccinia virus, or insect virus vectors such as baculovirus or Togavirus, may also be used.

The type of a promoter used in the present invention is not particularly limited, as long as it is a promoter compatible with a host used in expression of a gene.
When an animal cell is used as a host, for example, available promoters include an SRα promoter, a CMV promoter, an SV40 promoter, an LTR promoter, an HSV-TK promoter, an EF-1α promoter, and the like.
When Escherichia coil is used as a host, available promoters include a tac promoter, a trp promoter, a lac promoter, a recA promoter, a λPL promoter, a lpp promoter, a T7 promoter, and the like. When Bacillus subtilis is used as a host, available promoters include an SPO1 promoter, an SPO2 promoter, a penP promoter, and the like.
When yeast is used as a host, available promoters include a PHO5 promoter, a PGK promoter, a GAP promoter, and an ADH promoter, and the like.
When an insect cell is used as a host, a polyhedrin promoter, a P10 promoter, and the like are preferable.

To the recombinant vector of the present invention, not only a nucleic acid sequence encoding an Evl protein or a portion thereof and a promoter sequence, but also a selective marker, a terminator, an enhancer, a splicing signal, a poly(A) addition signal, a ribosome binding sequence (SD sequence), an SV40 replication origin (SV40ori), and the like can be ligated.
The type of a selective marker is not limited. A hygromycin resistance marker (Hyg^{r}), a dihydrofolate reductase gene (dhfr), an ampicillin resistance gene (Amp^{r}), a kanamycin resistance gene (Kan^{r}), a neomycin resistance gene (Neo^{r}, G418), and the like can be used.
Moreover, for the purpose of facilitating isolation and purification of a recombinant protein, a tag sequence used for purification, such as a His tag, an HA tag, or GST, can be fused on the N-terminal side, C-terminal side, etc. of a protein to be expressed or a portion thereof.
When the host is Escherichia coli, an alkaline phosphatase signal, an OmpA signal, and the like can be used. When the host is Bacillus subtilis, an α-amylase signal sequence, a subtilis signal sequence, and the like can be used. When the host is yeast, an α-factor signal sequence, an invertase signal sequence, and the like can be used. When the host is an animal cell, an insulin signal sequence, an α-interferon signal sequence, and the like can be used, for example.

DNA encoding an Evl protein or a portion thereof can be inserted into the aforementioned vector by adding the cloned DNA to a linker, directly or after digesting it with restriction enzymes as desired, and then incorporating the resultant DNA into the restriction site or multicloning site of vector DNA. The thus ligated DNA may have ATG acting as a translation start codon on the 5'-terminal side thereof and may also have TAA, TGA, or TAG acting as a translation stop codon on the 3'-terminal side thereof. Such translation start codon and translation stop codon may also be added to the DNA, using an appropriate synthetic DNA adapter. It is necessary that DNA to be ligated be incorporated into the vector, so that the polypeptide of the present invention encoded in the DNA can be expressed in a host cell.

In order to allow an Evl protein or a partial peptide thereof to express, it is necessary that a suitable host cell be transformed with an expression vector, into which a nucleic acid encoding such protein or partial peptide has been inserted. The type of a host used in such transformation is not particularly limited, as long as it allows the Evl protein to express therein. Examples of such a host include: bacteria belonging to genus Escherichia such as Escherichia coli, genus Bacillus such as Bacillus subtilis, genus Pseudomonas such as Pseudomonas putida, or genus Rhizobium such as Rhizobium meliloti; yeasts such as Saccharomyces cerevisiae, Shizosaccharomyces pombe, or Pichia pastoris; monkey cells such as COS-7 and Vero; Chinese hamster ovary cells (CHO cells); and insect cells such as Sf9 or Sf21.

As a method of introducing a recombinant vector into Escherichia coli, a method using calcium ion, an electroporation method, and the like can be used. As a method of introducing a recombinant vector into yeast, an electroporation method, a spheroplast method, a lithium acetate method, and the like can be used. As a method of introducing a recombinant vector into an animal cell or an animal cell, a DEAE-dextran method, an electroporation method, a calcium phosphate method, a method using cationic lipids, and the like can be used.

An Evl protein or a partial peptide thereof can be produced by culturing a transformant, allowing the protein or the partial peptide thereof to express in the culture of the transformant, and then isolating the protein or the partial peptide thereof from the culture. The term "culture" is used herein to mean all of a culture supernatant, a cultured cell, a cultured cell mass, and a disintegrated product of such cell or cell mass.
As a medium for culturing a transformant using a microorganism such as Escherichia coli or yeast as a host, either a natural medium or a synthetic medium may be used, as long as it contains a carbon source, a nitrogen source, inorganic salts, and the like that can be assimilated by microorganisms, and it is able to efficiently culture the transformant. Examples of a carbon source used herein include carbohydrates such as glucose, fructose, sucrose, or starch, organic acids such as acetic acid or propionic acid, and alcohols such as ethanol or propanol. Examples of a nitrogen source used herein include the ammonium salts of inorganic acids or organic acids, such as ammonia, ammonium chloride, ammonium sulfate, ammonium acetate, or ammonium phosphate, other nitrogen-containing compounds, peptone, meat extract, and corn steep liquor. Examples of inorganic salts used herein include monopotassium phosphate, dipotassium phosphate, magnesium phosphate, magnesium sulfate, sodium chloride, ferrous sulfate, manganese sulfate, copper sulfate, and calcium carbonate.

The culture is carried out under conditions that are suitable for host cells. For example, as a medium used for culturing Escherichia coli, an LB medium, an M9 medium, and the like are preferable. In order to allow a promoter to act efficiently, an agent such as isopropyl-1-thio-β-D-galactoside or 3β-indolylacrylic acid may be added, as desired. In the case of Escherichia coli, the culture is generally carried out at approximately 15°C to 37°C for approximately 3 to 24 hours, and thereafter, aeration or stirring may also be carried out, if necessary. In a case in which the host is Bacillus subtilis, the culture is generally carried out at approximately 30°C to 40°C for approximately 6 to 24 hours, and thereafter, aeration or stirring may also be carried out, if necessary.

As a medium for culturing yeast, an SD medium or a YPD medium may be used. The pH of the medium is preferably adjusted to pH 5 to 8. The culture is generally carried out at approximately 20°C to 35°C for approximately 24 to 72 hours, and thereafter, aeration or stirring may also be carried out, if necessary. When a transformant whose host is an insect cell or an insect is cultured, a Grace's insect cell culture medium that contains bovine serum and the like may be used. The pH of the medium is preferably adjusted to pH 6.2 to 6.4. The culture is generally carried out at approximately 27°C for approximately 3 to 5 days, and thereafter, aeration or stirring may also be carried out, if necessary.

When a transformant whose host is an animal cell is cultured, an MEM medium, a DMEM medium, an RPMI-1640 medium, or the like, which contains approximately 5% to 20% fetal bovine serum, is used. The pH is preferably pH 6 to 8. The culture is generally carried out at approximately 30°C to 40°C for approximately 15 to 60 hours, and thereafter, aeration or stirring may also be carried out, if necessary. An Evl protein or a partial peptide thereof may be separated and purified from the aforementioned culture by the following method, for example.

In order to extract such Evl protein or a partial peptide thereof from the cultured cell mass or the cultured cells, there may be applied, as appropriate, a method comprising collecting a cell mass or cells according to a known method after completion of the culture, suspending such cell mass or cells in a suitable buffer solution, and then disintegrating such cell mass or cells with the use of ultrasonic wave, lysozyme, and/or a freezing and thawing method, followed by centrifugation or filtration, so as to obtain a crude extract of the Evl protein or the partial peptide thereof. The buffer solution may comprise protein denaturants such as urea or guanidine hydrochloride, or surfactants such as Triton X-100. When the Evl protein or the partial peptide thereof is secreted into the culture solution, a culture supernatant is separated from a cell mass or cells according to a known method after completion of the culture, and such supernatant is then collected. The Evl protein or the partial peptide thereof contained in the thus obtained culture supernatant or extract may be purified by the appropriate combined use of known separation and purification methods. Known separation and purification methods that can be used herein include: methods utilizing solubility, such as a salting-out method and a solvent precipitation method; methods mainly utilizing a difference in molecular weight, such as a dialysis method, an ultrafiltration method, a gel filtration method, and SDS-PAGE; methods utilizing a difference in electric charge, such as ion exchange chromatography; methods utilizing specific affinity, such as affinity chromatography; methods utilizing a difference in hydrophobicity, such as reversed-phase high performance liquid chromatography; methods utilizing a difference in isoelectric point, such as an isoelectric focusing method; and other methods.

A composition comprising the Evl protein, the partial peptide thereof, or an expression vector containing such protein or peptide, can be used to cleave a single-stranded DNA and/or to bind the 5'-terminus of such single-stranded DNA to the 3'-terminus thereof. The present composition may comprise all substances necessary for the cleavage or binding of a single-stranded DNA, as well as the Evl protein, the partial peptide thereof, the expression vector therefor, and the like. For example, the present composition may comprise metal ion such as Mg²⁺, ATP, and auxiliary substances such as a buffer for keeping pH constant.

In another embodiment, the present invention provides a composition, which further comprises a Rad51B protein or an expression vector for the Rad51B protein, as well as the aforementioned active ingredients, auxiliary substances, and others.
The term "Rad51B protein" is used herein to mean a protein having an amino acid sequence identical to or substantially identical to the amino acid sequence shown in SEQ ID NO: 14, 16, or 18. The description "protein having an amino acid sequence ... substantially identical to ..." is used herein to mean a protein, which has an amino acid sequence showing amino acid identity of approximately 60% or more, preferably approximately 70% or more, more preferably approximately 80%, 81%, 82%, 83%, 84%, 85%, 86%, 87%, 88%, 89%, 90%, 91%, 92%, 93%, 94%, 95%, 96%, 97% or 98%, and most preferably approximately 99%, with the amino acid sequence shown in SEQ ID NO: 14, 16, or 18, and which has ATPase activity and activity of binding to a Holiday structure that is a DNA structure specific to homologous recombination.

Alternatively, the protein having an amino acid sequence substantially identical to the amino acid sequence shown in SEQ ID NO: 14, 16, or 18 may be a protein, which has an amino acid sequence comprising a deletion, substitution, or addition of one or several amino acids (preferably approximately 1 to 30, more preferably approximately 1 to 10, and further preferably 1 to 5 amino acids) with respect to the amino acid sequence shown in SEQ ID NO: 14, 16, or 18, and which has ATPase activity and activity of binding to a Holiday structure that is a DNA structure specific to homologous recombination.
The aforementioned deletion, addition and substitution of amino acids may be present in an isolated, native polypeptide. Otherwise, a gene encoding the protein of the present invention may be modified by a method known in the present technical field, so that such deletion, addition or substitution of amino acids may be newly introduced into a protein. For example, substitution of specific amino acid residue(s) may be carried out by substituting nucleotides with other nucleotides according to a known method such as a Gupped duplex method or a Kunkel method, or a method equivalent thereto, using a commercially available kit (for example, MutanTM-G (TAKARA), MutanTM-K (TAKARA), etc.).

The Rad51B protein may be obtained from native environment, or may be obtained by allowing a recombinant protein to express. When a recombinant Rad51B protein is allowed to express, an expression vector and components necessary for such expression may be selected in accordance with the above-described method for expressing an Evl protein. Such recombinant protein may be obtained in accordance with the aforementioned method for obtaining a recombinant Evl protein.

In addition, in a further embodiment, the present invention provides a composition, which further comprises a DNA topoisomerase type I protein or an expression vector for the DNA topoisomerase type I protein, as well as the Evl protein or the expression vector for the Evl protein. In general, the term "DNA topoisomerase type I" is also referred to as "TopoI." However, it is unnecessary to attach our mind to such common name. The DNA topoisomerase type I has activity of introducing a nick into one strand of a circular (double-stranded) DNA, passing the other stand through it, and then rebinding the nick. Thus, the DNA topoisomerase type I is considered to include all enzymes having activity of alleviating the supercoiling of DNA. Accordingly, the "DNA topoisomerase type I" of the present invention also includes those derived from either prokaryotes or eukaryotes. In addition, enzymes acting to relax either positive or negative supercoiling of DNA and enzymes acting to relax both positive and negative supercoiling of DNA are also included in the DNA topoisomerase type I of the present invention. A DNA topoisomerase type I protein may be obtained from native environment, or may be obtained by allowing a recombinant protein to express. Otherwise, commercially available products may be purchased. When a recombinant DNA topoisomerase type I protein is allowed to express, an expression vector and components necessary for such expression may be selected in accordance with the above-described method for expressing an Evl protein. Such recombinant protein may be obtained in accordance with the aforementioned method for obtaining a recombinant Evl protein.

In a further embodiment, the present invention provides a composition for inhibiting Evl activity. The Evl protein has activity of cleaving and/or binding a single-stranded DNA. When such Evl activity is suppressed or regulated, at the correct time, by a composition for inhibiting such activity, the formation of a single-stranded DNA having a more desired form can be achieved. Compounds contained as active ingredients in the composition for inhibiting Evl activity include aclarubicin, dequalinium, DIDS (disodium salt), β-rubromycin, and/or 3-ATA (3-amino-9-thio(10H)-acridone). These compounds may be contained in the composition for inhibiting Evl activity in the form of salts.

The composition of the present invention may further comprise additive substances that are generally necessary for preparation of reagents, such as a buffer used to adjust pH, salts used to adjust ionic strength, and a protease inhibitor, as well as active ingredients such as a compound, a protein, and DNA. Such additive substances may be appropriately selected and used by persons skilled in the art, depending on the intended use of the composition of the present invention.

In a further embodiment, the present invention provides a method for producing a single-stranded DNA molecular weight marker using the composition of the present invention, and a single-stranded DNA molecular weight marker produced by the aforementioned method. The single-stranded molecular weight marker of the present invention is produced by preparing single-stranded DNA molecules each having a different molecular weight that is equal to the integral multiple of the molecular weight of another single-stranded DNA having a known molecular weight. Accordingly, the molecular weight marker of the present invention is provided as a mixture of a single-stranded DNA used as a starting substance and/or single-stranded DNA molecules each having a different molecular weight that is equal to the integral multiple of the molecular weight of the aforementioned single-stranded DNA. Moreover, the molecular weight marker as such mixture is separated by agarose gel electrophoresis, and a single-stranded DNA corresponding to each molecular weight is cleaved, so that a single-stranded DNA having a specific molecular weight can be produced. Any type of single-stranded DNA can be used in the method for producing the single-stranded DNA molecular weight marker of the present invention. A circular single-stranded DNA is preferably used.
As described above, using a composition comprising the Evl protein of the present invention, a single-stranded DNA can be cleaved and can be then bound again. Thus, according to the present invention, single-stranded DNA molecules having various lengths (or molecular weights) can be produced. For example, referring to the example section of the present invention, a molecular weight marker consisting of 5368 × n nucleotides (wherein n represents an integer of 1 or greater) can be produced by allowing a composition comprising the Evl protein of the present invention to act on a φ×174 circular single-stranded DNA (5368 nucleotides), as shown in Figures 14a, 15a, 16a, 17a, and 18a. (In Figures 14a, 15a, 16a, 17a, and 18a, single-stranded DNA is schematically shown with oval shapes on the right side of a photograph of gel. A single oval shape indicates the position of a single-stranded DNA consisting of 5368 nucleotides, two oval shapes indicate the position of a single-stranded DNA consisting of 5368 × 2 nucleotides, and three oval shapes indicate the position of a single-stranded DNA consisting of 5368 × 3 nucleotides.) Similarly, using a single-stranded DNA having a different molecular weight, such as M13mp18 (7250 nucleotides), a molecular weight marker of 7250 × n (wherein n represents an integer of 1 or greater) can be produced (please refer to Figure 4).
Using the thus produced single-stranded DNA molecular weight marker, when a single-stranded DNA is extracted with helper phage or the like, the molecular weight of the extracted DNA can be examined.
Even after a single-stranded DNA marker produced with the composition of the present invention has been precipitated with ethanol and has been then freeze-dried, its molecular weight does not change. Thus, the present single-stranded DNA marker can be used as a stable single-stranded DNA molecular weight marker.

Examples will be given below. However, these examples are not intended to limit the scope of the present invention.

### Examples

### 1. Purification of human Evl protein

An Evl protein (NCBI accession No. AAF21709) DNA fragment (for example, SEQ ID NO: 1) was isolated from a human cDNA pool (Clontech) by a PCR method, and it was then cloned into the NdeI site of a pET15b vector (Novagen). In this construct, a His tag was fused on the N-terminal side of the isolated gene. The Evl protein was allowed to express using an E. coli BL21 (DE3) codon plus-RP strain (Stratagene), and it was then purified via 4 steps including a step of removing 6 × His tag. First, cells that expressed the Evl protein were suspended in buffer A (20 mM potassium phosphate (pH 8.5), 700 mM NaCl, 5 mM 2-mercaptoethanol, 10 mM imidazole, and 10% glycerol), and the cells were then disintegrated with an ultrasonic disintegrator. The obtained cell disintegrated solution was centrifuged at 30,000 × g at 4°C for 20 minutes, and the obtained supernatant was gently mixed with 8 ml of Ni-NTA agarose beads (QIAGEN). Thereafter, the obtained mixture was allowed to bind to the His tag-fused Evl protein (His-Evl) by a batch method at 4°C for 1 hour.

Evl-bound beads were washed with 80 ml of buffer B (20 mM potassium phosphate (pH 8.5), 700 mM NaCl, 5 mM 2-mercaptoethanol, 30 mM imidazole, and 10% glycerol). The beads were then washed with 80 ml of buffer C (20 mM potassium phosphate (pH 8.5), 700 mM NaCl, 5 mM 2-mercaptoethanol, 60 mM imidazole, and 10% glycerol), and were then washed with 80 ml of the buffer B again. Thereafter, an Econo-Column (Bio-Rad) was filled with the obtained Evl-bound beads. The thus filled Evl-bound beads were washed with 300 ml of buffer D (20 mM potassium phosphate (pH 8.5), 100 mM NaCl, 5 mM 2-mercaptoethanol, 30 mM imidazole, and 10% glycerol), and His-Evl was then eluted by linear concentration gradient elution with imidazole of 30 to 300 mM.

Buffer F (10 mM potassium phosphate (pH 8.5), 100 mM NaCl, 5 mM 2-mercaptoethanol, and 10% glycerol) was added in an equal amount to a fraction containing His-Evl, and further, the obtained mixture was gently mixed with 10 ml of hydroxyapatite (BIO-RAD) by a batch method at 4°C for 1 hour. Thereafter, resin was washed with 80 ml of buffer G (20 mM potassium phosphate (pH 8.5), 100 mM NaCl, 5 mM 2-mercaptoethanol, and 10% glycerol), and an Econo-Column was then filled with the resultant resin. The thus filled resin was washed with 300 ml of buffer H (10 mM potassium phosphate (pH 8.5), 225 mM NaCl, 5 mM 2-mercaptoethanol, and 10% glycerol), and His-Evl was then eluted by linear concentration gradient elution with NaCl of 225 to 1000 mM and potassium phosphate (pH 8.5) of 10 to 300 mM.
5 units of Thrombin Protease (GE Healthcare Bio-Sciences) was added per mg of the obtained His-Evl, and the obtained mixture was then dialyzed at 4°C against 4 L of buffer J (20 mM potassium phosphate (pH 8.5), 130 mM NaCl, 5 mM 2-mercaptoethanol, and 10% glycerol).

After removing the His tag, the Evl protein was further purified by Superdex200 gel filtration column (HiLoad 26/60 Superdex200 prep grade, GE Healthcare Bio-Sciences) chromatography. After completion of the purification, the Evl protein was dialyzed against buffer K (20 mM HEPES (pH 7.3), 100 mM NaCl, 5 mM 2-mercaptoethanol, and 30% glycerol) or against buffer L (20 mM potassium phosphate (pH 8.5), 700 mM NaCl, 5 mM 2-mercaptoethanol, and 30% glycerol). The resultant was preserved at -20°C. The concentration of the purified Evl protein was measured by a Bradford method using BSA as a standard. Figure 1a shows the results of the 12% SDS-PAGE of the purified Evl protein.

### 2. Pull-down assay using Evl- or Rad51B-bound beads

A Rad51B protein was purified in accordance with the previously reported method (Yokoyama et al., J. Biol. Chem. 278: 2767-2772, 2003).
An Evl protein and a Rad51B protein were each allowed to bind to Affi-Ge110 beads (Bio-Rad) in accordance with the instruction manual Ethanolamine (pH 8.0) was added thereto, so that the remaining ester residues had a final concentration of 100 mM. Thereafter, the obtained mixture was incubated at 4°C overnight. The Affi-Gel 10-Evl beads were washed 3 times with 500 µl of washing buffer 1 (20 mM potassium phosphate (pH 8.5), 30% glycerol, 700 mM NaCl, 5 mM 2-mercaptoethanol, and 0.05% Triton X-100). The Affi-Gel 10-Rad51B beads were washed 3 times with washing buffer 2 (20 mM HEPES-NaOH (pH 7.3), 30% glycerol, 90 mM NaCl, 2 mM 2-mercaptoethanol, 0.1% Triton X-100, 2 mM ammonium sulfate, and 0.1 mM EDTA). Thereafter, a 50% suspension of such Affi-Gel 10-protein was prepared, and it was then preserved at 4°C.

In order to carry out a binding assay, an Affi-Gel 10-protein suspension (30 µl) was incubated with 10 µg of an Evl or Rad51B protein at room temperature for 150 minutes. Affi-Gel 10-Evl beads were washed 4 times with 100 µl of buffer 1 (20 mM potassium phosphate (pH 8.5), 30% glycerol, 700 mM NaCl, 5 mM 2-mercaptoethanol, and 0.3% Triton X-100). Affi-Gel 10-Rad51B beads were washed 4 times with 100 µl of buffer 2 (20 mM HEPES-NaOH (pH 7.3), 30% glycerol, 90 mM NaCl, 2 mM 2-mercaptoethanol, 2 mM ammonium sulfate, 0.1 mM EDTA, and 0.35% Triton X-100). An SDS-PAGE sample treatment buffer (2 ×) was directly mixed with the washed beads, and the obtained mixture was then subjected to a heat treatment at 100°C for 2 minutes. The reaction product was separated by 12% SDS-PAGE, and the protein was then stained Coomassie Brilliant Blue.

As shown in Figure 1b, the Rad51B protein was pulled downed by the Evl-bound beads. In addition, the Evl protein was pulled down by the Rad51B-bound beads (Figure 1c).
From these results, it became clear that the Evl protein directly binds to Rad51B. When such Evl-bound beads were used in a pull-down assay, Rad51B bound to the Evl-bound beads at a stoichiometric ratio of 1 : 1 (Figure 1b). On the other hand, a large amount of Evl protein was precipitated together with the Rad51B-bound beads (Figure 1c). Accordingly, it is considered that an Evl protein is polymerized with another Evl protein to form a complex. As a result of gel filtration analysis, the Evl protein was eluted at a fraction of approximately 600 kDa, and thus it was demonstrated that the Evl protein formed an approximately 13-mer multimer (Figure 2).

### 3. DNA binding assay

Subsequently, the binding ability of the Evl protein to DNA was analyzed. The Evl protein was mixed with a φ × 174 circular single-stranded DNA (40 µM) or a φ × 174 supercoiled double-stranded DNA (10 µM) in 10 µl of a reaction solution (20 mM HEPES (pH 7.5), 1 mM DTT, 1 mM MgCl₂, and 100 mg/ml BSA), and the mixture was then reacted at 37°C for 15 minutes. The reaction product was analyzed by 0.8% agarose gel electrophoresis (3 V/cm, 3 hours) using a 1 × TAE (40 mM Tris-acetate and 1 mM EDTA) buffer. A band of DNA was stained with ethidium bromide (Figure 3). As a result of the assay, it was found that the Evl protein efficiently binds to a single-stranded DNA and a supercoiled double-stranded DNA (Figure 3).

### 4. Assay of catenation of single-stranded DNA

A φ × 174 or M13mp18 circular single-stranded DNA (20 µM) was incubated at 37°C in 20 mM HEPES-NaOH (pH 7.5), 1 mM DTT, 1 mM MgCl₂, and 0.1 mg/ml BSA. Thereafter, the sample was subjected to a deproteination treatment with 0.2% SDS and 1.3 mg/ml proteinase K. The obtained product was separated by 0.9% agarose gel electrophoresis, and a DNA band was then stained with SYBR Gold (Invitrogen).
After the Evl protein had been allowed to act on the aforementioned DNA molecules, the circular single-stranded DNA formed a multimer (Figure 4a, lanes 2 and 4; and Figure 4b, lane 2), but the supercoiled double-stranded DNA was not particularly changed (Figure 4b, lane5). The Evl protein did not induce a change in the topology of the supercoiled double-stranded DNA. Thus, it is considered that the activity of the Evl protein differs from the activity of topoisomerase I (Figure 4b, lane 6).

Subsequently, the metal ion requirement of the Evl protein was examined. As a result, it was found that the Evl protein strongly requires Mg²⁺ (Figure 5a). Even in the absence of Mg²⁺, the catenation activity of the Evl protein was observed. However, such activity was promoted by the presence of Mg²⁺. The optimal concentration of Mg²⁺ depends on reaction conditions (the concentrations of other ingredients, such as a glycerol concentration or a salt concentration). The concentration of Mg²⁺ is, for example, 0.1 mM to 20 mM, preferably 0.5 mM to 15 mM, more preferably 0.5 mM to 10 mM, and further preferably 0.5 mM to 2 mM. In addition, with regard to metal ions other than Mg²⁺, Ca²⁺ exhibited the same effect (Figure 5b). Under the present experimental conditions, an enormous DNA complex-like product was formed in the case of adding Mn²⁺. In addition, Zn²⁺ did not have the same level of activity of promoting the activity of the Evl protein as that of Mg²⁺ (Figure 5b).
Moreover, the formed single-stranded DNA multimer was not eliminated, even after it had been treated at 100°C for 10 minutes (Figure 6). Thus, it is suggested that such multimer be a catemer of circular single-stranded DNA molecules catenated to one another to form a ring.
Subsequently, in order to visually understand a state in which circular single-stranded DNA molecules were catenated by the action of the Evl protein, a generated product was observed under an electron microscope.

### 5. Electron microscope observation

A single-stranded DNA catemer formed by the Evl protein was extracted by a phenol/chloroform method, and it was then recovered by ethanol precipitation. Subsequently, such single-stranded DNA catemer was coated with a RecA protein (New England Biolabs) in the absence of ATP, and it was then stained with 2% uranyl acetate on a carbon-coated copper grid. The stained sample was visualized by a rotary shadow method using tungsten, and it was then observed under a JEOL JEM2000 FX electron microscope.
As shown in Figure 7, a circular single-stranded DNA catemer containing 2 or 3 single-stranded DNA molecules was observed.

As stated above, as a result of the assay of catenation of single-stranded DNA molecules and the observation under an electron microscope, it is considered that the Evl protein cleaves a circular single-stranded DNA and then binds the 5'-terminus thereof to the 3'-terminus thereof, so as to form a catemer. Accordingly, it can be concluded that the Evl protein has activity of cleaving a single-stranded DNA and binding the 5'-terminus of such single-stranded DNA to the 3'-terminus thereof.
It is to be noted that it has been revealed that such catenation occurs even in the case of linear single-stranded DNA molecules.

### 6. Influence of Rad51B

Subsequently, the influence of Rad51B on the catenation of single-stranded DNA molecules by the Evl protein was examined. As shown in Figure 8a, it was found that the catenation of DNA molecules by the Evl protein was significantly promoted by Rad51B in the presence of ATP (lane 4). In contrast, such Rad51B-dependent promotion of catenation was not significant in the absence of ATP (Figure 8a, lane 8). Accordingly, it is suggested that the ATP-bound Rad51B protein would promote the catenation of DNA molecules by the Evl protein. The effect of Rad5lB to promote the activity of the Evl protein increases in an added Rad51B concentration dependent manner. However, when Rad51B was added in a concentration of 1 µM or higher to a 4 µM Evl protein, such promoting effect was not increased any more (Figure 9).
On the other hand, a Rad51 protein showing sequence similarity to Rad51B inhibited the catenation of DNA molecules by the Evl protein, regardless of the presence or absence of ATP (Figure 8b). These results suggest that the activity of the Evl protein should be promoted by specific functional interaction between the Evl and Rad51B proteins.

### 7. EVH2 domain of Evl protein

In order to identify a functional domain that causes the catenation of single-stranded DNA molecules by the Evl protein, two Evl fragments comprising amino acid residues at positions 1-221 and at positions 222-418, namely, Evl (1-221) and Evl (222-418), were purified. Purification of Evl (1-221) and Evl (222-418) was carried out in accordance with the protocols used in purification of the entire-length Evl protein.
Constructs used in expression of Evl (1-221) and Evl (222-418) were produced according to an ordinary method. Thereafter, Escherichia coli (BL21(DE3)) was transformed with each construct, so as to allow it to express each protein. With regard to Evl (1-221), a cell extract was recovered, it was then passed through a Ni-NTA agarose column (Invitrogen), and the obtained peak fraction was then mixed with hydroxyapatite (Bio-Rad). After the mixture had been centrifuged, a supernatant that had not bound to the hydroxyapatite was recovered, and a His tag was removed by a thrombin treatment. Thereafter, Evl (1-221) was separated from the tag by Superdex200 (GE Healthcare) column chromatography. The Evl (1-221) obtained by Superdex200 column chromatography was used in the subsequent experiment (Figure 10). On the other hand, with regard to Evl (222-418), a cell extract was recovered, and it was then passed through a Ni-NTA agarose column (Invitrogen). Thereafter, the obtained peak fraction was passed through hydroxyapatite (Bio-Rad), so that a peak fraction was recovered. A His tag was removed from the recovered Evl (222-418) by a thrombin treatment. Subsequently, the Evl (222-418) was separated from the tag by Superdex200 (GE Healthcare) column chromatography. The Evl (222-418) obtained by Superdex200 column chromatography was used in the subsequent experiment (Figure 11).

An Evl (1-221) mutant comprises EVH1 and a proline-rich domain, whereas an Evl (222-418) mutant comprises an EVH2 domain. As shown in Figure 12b, the Evl (222-418) mutant exhibited ssDNA catenation activity (lane 4), but the Evl (1-221) mutant did not exhibit such ssDNA catenation activity (lane 3). In addition, the activity of the Evl (222-418) mutant was promoted by the Rad51B protein (Figure 12c, lane 8). In contrast, in the case of the Evl (1-221) mutant, although the Ray51B protein was added thereto, no activity was detected (Figure 12c, lane 6).
Accordingly, it is considered that the EVH2 domain is necessary for the ssDNA catenation activity of the Evl protein.

### 8. Effect of promoting catenation of single-stranded DNA molecules caused by coexistence of DNA topoisomerase type I protein and Evl protein

A 20 µM φ × 174 single-stranded DNA, a 4 µM Evl protein, and a DNA topoisomerase type I protein in each concentration or unit number (derived from Escherichia coli or a human) were added to 10 µl of a reaction solution (20 mM HEPES, 1 mM DTT, 100 µg/ml BSA, and 1 mM MgCl₂). The obtained mixture was reacted at 37°C for 1 hour. Thereafter, the sample was subjected to a deproteination treatment using 0.2% SDS and 1.3 mg/ml proteinase K. The obtained product was separated by 0.9% agarose gel electrophoresis, and a DNA band was then stained with SYBR Gold (Invitrogen) (Figure 13).
As a result, it was revealed that the catenation of single-stranded DNA molecules is promoted in the coexistence of the Evl protein and the DNA topoisomerase type I protein derived from Escherichia coli (Figure 13a, lanes 4-5) or derived from a human (Figure 13b, lane 4).

### 9. Searching for low molecular weight compound that affects single-stranded DNA catenation activity of Evl protein

A low molecular weight compound that can be used to regulate at the correct time right the single-stranded DNA catenation activity of the Evl protein was searched. As a result, aclarubicin, dequalinium, DIDS, β-rubromycin, and 3-ATA were discovered as such compounds.
The inhibitory activity of each of the above compounds was detected as follows.
With regard to the influence of each compound on the catenation activity of the Evl protein (Figures 14a, 15a, 16, 17a, and 18a), each compound in final concentrations of 1, 5, 10, and 20 µM, a φ × 174 single-stranded DNA in a final concentration of 20 µM, and an Evl protein in a final concentration of 4 µM were added to 10 µl of a reaction solution (20 mM HEPES, 1 mM DTT, 100 µg/ml BSA, and 1 mM MgCl₂). The obtained mixture was reacted at 37°C for 1 hour. After completion of the reaction, 2 µl of a PK solution (0.2% SDS and 1.3 mg/ml proteinase K) was added to the reaction solution, and the obtained mixture was then reacted at 37°C for 15 minutes. Thereafter, the reaction product was electrophoresed on a 0.8% HGT agarose gel, and DNA was then detected with SYBR Gold.
Moreover, in a gel shift method involving single-stranded DNA binding activity (Figures 14b, 15b, 16b, 17b, and 18b), each compound in final concentrations of 1, 5, 10, and 20 µM, a φ × 174 single-stranded DNA in a final concentration of 20 µM, and an Evl protein in a final concentration of 0.3 µM were added to 20 µl of a reaction solution (20 mM HEPES, 1 mM DTT, 100 µg/ml BSA, and 1 mM MgCl₂). The obtained mixture was reacted at 37°C for 15 minutes. Thereafter, the reaction product was electrophoresed on a 0.8% HGT agarose gel, and DNA was then detected with ethidium bromide.

### 9-1.Aclarubicin

Aclarubicin is an anthracycline antitumor agent, cardiotoxicity of which has been significantly decreased. It is also an agent for inhibiting the catalytic activity of topoisomerase I/II. Moreover, aclarubicin suppresses the chymotrypsin-like activity of 20S proteasome, so that it also suppresses the decomposition of a ubiquitinated protein. Such aclarubicin has been known to inhibit IL-1β-induced iNOS generation in aorticsmooth muscle cells. Aclarubicin is used on trial as an antitumor antibiotic (product name: Aclacinon) at clinical sites. This agent binds to the DNA of a cancer cell to strongly inhibit nucleic acid synthesis and RNA synthesis, and thus it is used for the purpose of alleviating and improving the subjective and objective symptoms of stomach cancer, lung cancer, breast cancer, malignant lymphoma, and acute leukemia. In the present invention, it was discovered that such aclarubicin inhibits the activity of an Evl protein to catenate single-stranded DNA molecules (Figure 14a). Furthermore, the influence of aclarubicin on the activity of the Evl protein to bind to a single-stranded DNA was examined by a gel shift assay method (Figure 14b). As a result, it was found that aclarubicin does not change the activity of the Evl protein to bind to circular single-stranded DNA. From the results of the aforementioned analysis, it became clear that aclarubicin inhibits the single-stranded DNA catenation activity of the Evl protein, without inhibiting the single-stranded DNA binding activity of the same protein.

### 9-2. Dequalinium

Dequalinium (dequalinium chloride) is an antitumoral, PKC-inhibitory agent. When UV is applied to dequalinium, such dequalinium covalently binds to PKCa or PKCβ so as to irreversibly inhibit them. Dequalinium is a strong, selective non-peptide blocker to an apamin-sensitive low transferable Ca²⁺-activated K⁺ channel, and it blocks neurotransmission. Moreover, it is accumulated selectively in the mitochondria of cancer cells, so that it inhibits energy production. Such dequalinium is used with a product name, "Nodoman Troche," at clinical sites. Since this agent acts on the proteins of bacteria and kills bacteria existing in the mouth or throat, it is used for prevention of infection such a spharyngitis, tonsillitis, stomatitis, and oral wound including wound of tooth extraction. In the present invention, it was found that dequalinium inhibits the single-stranded DNA catenation activity of the Evl protein (Figure 15a). Furthermore, the influence of dequalinium on the activity of the Evl protein to bind to a single-stranded DNA was examined by a gel shift assay method (Figure 15b). As a result, it was found that dequalinium changes the binding manner of the Evl protein that binds to a single-stranded DNA. From the results of the aforementioned analysis, it is considered that dequalinium changes the binding manner of the Evl protein that binds to a single-stranded DNA, and that it inhibits the activity of the Evl protein to catenate a circular single-stranded DNA.

### 9-3. DIDS (Disodium Salts)

DIDS is an anion transport inhibitor that inhibits Cl incorporation into neuroblastoma cells, and it exhibits antiulcer action. In addition, DIDS is also known to inhibit ATP transport into an endoplasmic reticulum. In the present invention, it was found that DIDS inhibits the single-stranded DNA catenation activity of the Evl protein (Figure 16a). Moreover, the influence of DIDS on the activity of the Evl protein to bind to a single-stranded DNA was examined by a gel shift assay method (Figure 16b). As a result, it was found that DIDS inhibits the single-stranded DNA binding activity of the Evl protein. From the results of the aforementioned analysis, it is considered that DIDS inhibits the single-stranded DNA binding activity of the Evl protein, so as to inhibit the single-stranded DNA catenation activity of the Evl protein.

### 9-4. β-rubromycin

β-rubromycin is an inhibitory agent for HIV reverse transcriptase. In addition, β-rubromycin is also known as an inhibitory agent for telomerase. In the present invention, it was found that β-rubromycin inhibits the single-stranded DNA catenation activity of the Evl protein (Figure 17a). Moreover, the influence of β-rubromycin on the activity of the Evl protein to bind to a single-stranded DNA was examined by a gel shift assay method (Figure 17b). As a result, it was found that β-rubromycin changes the binding manner of the Evl protein that binds to a single-stranded DNA. From the results of the aforementioned analysis, it is considered that β-rubromycin changes the binding manner of the Evl protein that binds to a single-stranded DNA, and that it inhibits the single-stranded DNA catenation activity of the Evl protein.

### 9-5. 3-ATA (3-amino-9-thio(10H)-acridone)

3-ATA is a CDK4 inhibitory agent. In addition, it is also found that 3-ATA suppresses the growth of p-16 mutation tumor. In the present invention, it was found that 3-ATA inhibits the single-stranded DNA catenation activity of the Evl protein (Figure 18a). Moreover, the influence of 3-ATA on the activity of the Evl protein to bind to a single-stranded DNA was examined by a gel shift assay method (Figure 18b). As a result, it was found that 3-ATA does not change the single-stranded DNA binding activity of the Evl protein. From the results of the aforementioned analysis, it was found that 3-ATA inhibits the single-stranded DNA catenation activity of the Evl protein, without inhibiting the single-stranded DNA binding activity of the Evl protein.

### Industrial Applicability

Since the composition of the present invention has both activity of cleaving a single-stranded DNA and activity of binding the 5'-terminus of such single-stranded DNA to the 3'-terminus thereof, it can be used as an effective tool for manipulating a single-stranded DNA in a genetic engineering manner. Moreover, using the composition of the present invention, it also becomes possible to provide a single-stranded DNA molecular weight marker and the like. Thus, it can be anticipated that the composition of the present invention will greatly contribute to the progression of research and development in the biological and medical fields.

## Claims

1. A composition for cleaving a single-stranded DNA and/or binding the 5'-terminus of such single-stranded DNA to the 3'-terminus thereof, which comprises an Evl protein described in the following (a) or (b):
(a) a protein having the amino acid sequence shown in SEQ ID NO: 2, 4, 6, 8, 10, 12, or 20; or
(b) a polypeptide, which has an amino acid sequence comprising a substitution, deletion, or insertion of one or several amino acids with respect to the amino acid sequence shown in SEQ ID NO: 2, 4, 6, 8, 10, 12, or 20, and which has activity of cleaving a single-stranded DNA and binding the 5'-terminus of such single-stranded DNA to the 3'-terminus thereof.

2. The composition according to claim 1, wherein said composition further comprises Mg²⁺ or Ca²⁺.

3. The composition according to claim 2, wherein a concentration of said Mg²⁺ is between 0.5 mM and 2.0 mM.

4. The composition according to any one of claims 1 to 3, wherein said composition further comprises a Rad51B protein and ATP.

5. The composition according to claim 4, wherein the molar ratio of said Evl protein to said Rad51B is from 1 : 0.5 to 1 : 4.

6. The composition according to claim 4 or 5, wherein a concentration of said ATP is between 0.5 mM and 2.0 mM.

7. The composition according to any one of claims 1 to 3, wherein it further comprises a DNA topoisomerase type I protein.

8. A composition for cleaving a single-stranded DNA and/or binding the 5'-terminus of such single-stranded DNA to the 3'-terminus thereof, which comprises a recombinant vector comprising a nucleic acid described in the following (a) or (b):
(a) a nucleic acid having the nucleotide sequence shown in SEQ ID NO: 1, 3, 5, 7, 9, 11, or 19; or
(b) a nucleic acid, which hybridizes under stringent conditions with the complementary strand of the nucleic acid having the nucleotide sequence shown in SEQ ID NO: 1, 3, 5, 7, 9, 11, or 19, and which encodes a polypeptide having activity of cleaving a single-stranded DNA and binding the 5'-terminus of such single-stranded DNA to the 3'-terminus thereof.

9. A composition for cleaving a single-stranded DNA and/or binding the 5'-terminus of such single-stranded DNA to the 3'-terminus thereof, which comprises a recombinant vector comprising a nucleic acid described in the following (a) or (b):
(a) a nucleic acid encoding a polypeptide having the amino acid sequence shown in SEQ ID NO: 2, 4, 6, 8, 10, 12, or 20; or
(b) a nucleic acid encoding a polypeptide, which has an amino acid sequence comprising a substitution, deletion, or insertion of one or several amino acids with respect to the amino acid sequence shown in SEQ ID NO: 2, 4, 6, 8, 10, 12, or 20, and which has activity of cleaving a single-stranded DNA and binding the 5'-terminus of such single-stranded DNA to the 3'-terminus thereof.

10. A method for producing a single-stranded DNA marker by reacting the composition according to any one of claims 1 to 9 with a single-stranded DNA.

11. A single-stranded DNA marker produced by the method according to claim 10.

12. A composition for inhibiting Evl protein activity, which comprises one or multiple compounds selected from the compound group consisting of aclarubicin, dequalinium, DIDS, β-rubromycin, and 3-ATA.
